(19) Europäisches Patentamt
European Patent Office
Office européen des brevets

(11) Numéro de publication: **0 246 982 B1**

(12) **FASCICULE DE BREVET EUROPEEN**

(45) Date de publication de fascicule du brevet: **03.02.93**

(51) Int. Cl.[5]: **C07D 405/06**, A01N 43/50, A01N 43/653, C07D 307/20, C07D 303/22, C07C 33/03, C07C 31/22

(21) Numéro de dépôt: **87420102.3**

(22) Date de dépôt: **17.04.87**

Le dossier contient des informations techniques présentées postérieurement au dépôt de la demande et ne figurant pas dans le présent fascicule.

# (54) Composés à groupements triazole ou imidazole et tétrahydrofurane utilisation de cuex-ci à titre de fongicides et procédés de préparation.

(30) Priorité: **23.04.86 FR 8606075**
**06.03.87 FR 8703281**

(43) Date de publication de la demande:
**25.11.87 Bulletin 87/48**

(45) Mention de la délivrance du brevet:
**03.02.93 Bulletin 93/05**

(84) Etats contractants désignés:
**AT BE CH DE ES FR GB GR IT LI LU NL SE**

(56) Documents cités:
**EP-A- 0 089 100**
**EP-A- 0 121 979**
**EP-A- 0 151 084**

(73) Titulaire: **RHONE-POULENC AGROCHIMIE**
**14-20, rue Pierre Baizet**
**F-69009 Lyon(FR)**

(72) Inventeur: **Greiner, Alfred**
**31, Chemin des Aulnes**
**F-69570 Dardilly(FR)**
Inventeur: **Pepin, Régis**
**27, Montée Castellane**
**F-69140 Rillieux-la-Pape(FR)**

(74) Mandataire: **Brachotte, Charles et al**
**RHONE-POULENC AGROCHIMIE DPI BP 9163**
**F-69263 Lyon Cedex 09(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 246 982 B1

## Description

La présente invention concerne de nouveaux composés, à usage phytosanitaire, à groupements triazole ou imidazole et tétrahydrofuranne. Elle concerne également les procédés de préparation desdits composés et les produits éventuellement utilisables à titre d'intermédiaires dans les procédés de préparation. Elle concerne ensuite l'utilisation à titre de fongicides de ces composés, les compositions fongicides à base de ces composés et les procédés pour lutter contre les maladies fongiques des cultures utilisant ces composés.

De nombreux produits à groupes triazole, notamment des fongicides, sont déjà connus en particulier par la demande de brevet européen n° 151 084. Cette demande de brevet décrit effectivement les composés dont la formule est indiquée en fin de description et leur utilisation comme fongicide.

Un but de la présente invention est de proposer des composés présentant des propriétés améliorées dans le traitement des maladies fongiques.

Un autre but de la présente invention est de proposer des composés présentant un spectre d'utilisation également amélioré dans le domaine des maladies fongiques.

Il a maintenant été trouvé que ces buts pouvaient être atteints grâce aux produits de l'invention. Ceux-ci sont caractérisés en ce qu'ils répondent à la formule (I) dans laquelle

A est le groupement (Y)n Ph-$(CH_2)$m où Ph est un noyau phénylène et m = 0 ou 1, de préférence 0,

Y est un atome d'halogène ou un groupe cyano ou nitro, ou un groupe alkyle ou alkoxy éventuellement halogénés,

n est un nombre entier positif ou nul, inférieur à 6, les groupements Y pouvant être identiques ou différents lorsque n est plus grand que 1,

Tr représente un groupe 1, 2, 4-triazole 1-yl et Im un groupe 1,3-imidazole 1-yl,

- $R_1$ à $R_5$, identiques ou différents, représentent l'atome d'hydrogène ou un radical alkyle comportant au plus six atomes de carbone (linéaire ou ramifié), cycloalkyle comportant au plus six atomes de carbone phényle, benzyle, alkoxy comportant au plus six atomes de carbone, ces divers radicaux pouvant être éventuellement substitués (par exemple par un ou plusieurs atomes ou radicaux tels que les atomes d'halogènes, et les radicaux alkoxy comportant au plus six atomes de carbone,
- $X_1$, $X_2$, identiques ou différents, représentent l'atome d'hydrogène, un atome d'halogène, un radical alkyle comportant au plus six atomes de carbone, un radical cycloalkyle comportant au plus six atomes de carbone, un radical phényle, ou benzyle, ces divers radicaux pouvant être éventuellement substitués de façon similaires aux groupes $R_1$ à $R_5$,

  un groupement Q-$R_6$ dans lequel Q représente O ou S, et $R_6$ représente l'atome d'hydrogène ou un radical alkyle comportant au plus six atomes de carbone, un radical cycloalkyle comportant au plus six atomes de carbone, phényle, benzyle, acyle, benzoyle,

  ces radicaux pouvant être substitués de façon similaire aux groupes $R_1$ à $R_5$ et pouvant avoir des significations identiques ou différentes lorsque $X_1$ et $X_2$

  correspondent chacun à $OR_6$ ou $SR_6$,

  un groupement de formule -Q-$R_7$-Q- où Q a la même signification que précédemment et dans laquelle $R_7$ est un radical divalent hydrocarboné, comprenant de 2 à 6 atomes de carbones, l'un de ces atomes de carbone pouvant être remplacé par un atome d'oxygène ou d'azote, ledit radical unique divalent étant lui-même éventuellement substitué (par exemple par un ou plusieurs atomes d'halogène ou radicaux alkyle comportant au plus six atomes de carbone, alkoxy comportant au plus six atomes de carbone, hydroxy), un groupement -N $R_8R_9$ dans lequel

  $R_8$ et $R_9$, identiques ou différents,

  représentent l'atome d'hydrogène ou un radical alkyle comportant au plus six atomes de carbone, un radical cycloakyle comportant au plus six atomes de carbone, phényle,

  ces divers radicaux pouvant être éventuellement substitués de façon similaire aux groupes $R_1$ à $R_5$,

  ou bien $R_8$ et $R_9$ peuvent former ensemble un radical unique divalent hydrocarboné, comprenant de 2 à 6 atomes de carbones, l'un de ces atomes de carbone pouvant être remplacé par un atome d'oxygène, de soufre ou d'azote, ledit radical unique divalent étant lui-même éventuellement substitué (par exemple par un ou plusieurs atomes d'halogène ou radicaux alkyle comportant au plus six atomes de carbone, alkoxy comportant au plus six atomes de carbone éventuellement halogénés, hydroxy),

  un groupement = N-$R_{10}$ dans lequel $R_{10}$ correspond à un radical alkyle comportant au plus six atomes de carbone, un radical cycloalkyle comportant au plus six atomes de carbone, à un groupement de formule $OR_{11}$ dans laquelle $R_{11}$ est un alkyle, ces radicaux pouvant être substitués de

façon similaire aux groupes $R_1$ à $R_5$, à un groupement hydroxy
un groupement $N_3$ à la condition que $X_2$ soit différent de $N_3$,
$X_1$ et $X_2$ peuvent constituer ensemble = 0 ou = S, aux conditions générales que $X_1$ et $X_2$ ne puissent pas correspondre simultanément à l'atome d'hydrogène, à un radical alkyle comportant au plus six atomes de carbone, un radical cycloalkyle comportant au plus six atomes de carbone, un radical phényle ou un radical benzyle et si $X_1$ et $X_2$ sont différents et que l'un des deux est $OR_6$ alors, dans ce cas là seulement, $R_6$ ne peut être que le groupe benzoyle ou acyle.

L'invention concerne également les formes salifiées des composés selon l'invention. Les formes salifiées sont les formes acceptables en agriculture parmi lesquelles on peut citer : les chlorhydrate, sulfate, oxalate, nitrate ou arylsulfonate ainsi que les complexes d'addition de ces composés avec des sels métalliques, et notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Il faut noter que les composés revendiqués ne sont pas décrits dans la demande de brevet européen n° 151 084.

Au sens du présent texte, on entend que l'adjectif inférieur, lorsqu'il qualifie un radical organique, signifie que ce radical comporte au plus six atomes de carbone. Ce radical peut être linéaire ou ramifié.

Le déposant souhaite souligner que les planches annexes ne sauraient en aucune manière être considérées comme des dessins mais comme faisant partie intégrante de la description de l'invention.

Les composés de formule I et les composés éventuellement utilisables à titre d'intermédiaires dans les procédés de préparation et qui seront définis à l'occasion de la description de ces procédés peuvent exister sous une ou plusieurs formes d'isomères selon le nombre de centres asymétriques de la molécule. L'invention concerne donc aussi bien tous les isomères optiques que leurs mélanges racémiques et les diastéréoisomères correspondants. La séparation des diastéréoisomères et/ou des isomères optiques peut s'effectuer selon les méthodes connues en soi. +

En vue des applications fongicides il a été trouvé que l'invention concernait de préférence les composés de formule I dans laquelle Y est un halogène et n = 1, 2 ou 3.

Il a été également trouvé qu'il était préférable d'utiliser les composés de formule I dans laquelle n = 1 ou 2, et Y est un atome d'halogène placé en ortho et/ou en para.

De préférence encore n = 2 et Y est un atome d'halogène, avantageusement le chlore placé en ortho et en para.

Compte tenu des restrictions définies ci-dessus prises séparemment ou en combinaisons, il a été trouvé qu'il était préférable en raison des propriétés fongicides d'utiliser les composés triazole de formule II dans laquelle $R_1$ à $R_5$ correspondent à un atome d'hydrogène, à un groupement alkyle ou cycloalkyle inférieur, Y, n, m ont la même signification que dans la formule I avec les variantes préférées énoncées ci-dessus, Hal correspond à un atome d'halogène, de préférence le chlore ou le brome, $X_1$ est un atome d'halogène, de préférence le chlore ou le brome, ou un atome d'hydrogène et avantageusement l'atome d'hydrogène.

Compte tenu des restrictions définies ci-dessus prises séparemment ou en combinaisons, il a été trouvé qu'il était préférable en raison des propriétés fongicides d'utiliser les composés triazole de formule III dans laquelle $R_1$ à $R_5$ correspondent à un atome d'hydrogène, à un groupement alkyle ou cycloalkyle inférieur, Y, n, m ont la même signification que dans la formule I avec les variantes préférées énoncées ci-dessus $X_1$ correspond à un atome d'hydrogène, à un groupement alkyle ou cycloalkyle inférieur, Q a la même signification que dans la formule I, $R^6$ correspond à un atome d'hydrogène, à un groupement alkyle ou cycloalkyle inférieur.

Les composés triazole de formule II préférés sont ceux dont les groupements $R_1$ à $R_5$ correspondent à un atome d 'hydrogène.

Les composés triazole de formule III préférés sont ceux dont les groupements $R_1$ à $R_5$ correspondent à un atome d'hydrogène où $X_1$ correspond à l'atome d'hydrogène ou alkyle ou cycloalkyle inférieur, et où $R_6$ correspond à un groupement alkyle, cycloalkyle inférieur et de préférence Q = 0.

La présente invention concerne également des procédés de préparation des composés selon l'invention.

Dans le cas où le groupement $X_2$ correspond à un atome d'halogène, (composé de formule II), un procédé de préparation consiste dans les étapes suivantes :

Etape a) On fait réagir une halogènocétone de formule IIa obtenue par un procédé connu, dans laquelle $\overline{Y, n, R_5}$ ont la même signification que celle donnée pour les composés de formule I et Z correspond à un atome d'halogène, avec un organométallique de formule IIb, dans laquelle $R_1$ à $R_4$, $X_1$, ont la même

signification que ci-dessus et M correspond à un métal alcalin ou à un magnésien (Mg Hal) ou à un zincique (Zn Hal) par exemple, dans un solvant, choisi de préférence parmi les éthers, tels que l'éther diéthylique ou le tétrahydrofuranne, les hydrocarbures aliphatiques, alicycliques ou aromatiques tels que l'hexane, le toluène à une température choisie entre -50 °C et le reflux du solvant considéré et dans un rapport molaire II a : II b compris de préférence entre 1,1 et 0,2. La réaction conduit au composé de formule IIc après neutralisation du milieu réactionnel.

Etape b) On fait réagir ensuite le composé de formule IIc avec un imidazole ou un triazole non substitué en présence de base organique ou minérale par exemple la pyrydine, la triéthylamine la soude, la potasse, les carbonates et bicarbonates de métaux alcalins ou alcalino terreux et dans un solvant approprié tel que par exemple les alcools, les cétones, les amides, les nitriles, les hydrocarbures aromatiques éventuellement halogénés à une température comprise entre 80° et le reflux du solvant et dans un rapport molaire IIc : imidazole ou triazole de préférence compris entre 1,1 et 0,2, ce qui conduit au composé de formule IId. La réaction passe en général par un intermédiaire époxyde de formule IIh qui peut éventuellement être isolé ou préparé séparémment par des méthodes connues de l'homme de l'art.

Etape c) On additionne sur le composé IId de préférence mole à mole une molécule d'halogène ou d'halogène mixte dans un solvant inerte tel que les hydrocarbures saturés ou aromatiques éventuellement halogénés ce qui conduit au composé IIe.

Etape d) Le composé II est obtenu de préférence à température ambiante par cyclisation du composé IIe en présence d'une base organique ou minérale indiquée en b dans un rapport molaire composé II : base de préférence compris entre 1,1 et 0,66. La réaction peut s'effectuer en milieu solvant protique ou aprotique (eau, alcool, cétone, nitrile, ester, hydrocarbure saturé ou aromatique éventuellement halogéné, diméthylsulfoxyde, amide comme le diméthylformamide).

Un second procédé de préparation des composés où le groupement $X_2$ est un halogène consiste à placer l'étape b) de greffage du noyau imidazole ou triazole après l'étape d) en utilisant le même mode opératoire pour les différentes étapes. Ainsi on additionne sur le composé IIc une molécule d'halogène ou d'halogènure d'halogène (halogène mixte) pour donner le composé IIf, celui-ci étant ensuite cyclisé pour donner le composé IIg, qui est ensuite pourvu d'un groupement triazole ou imidazole pour donner le composé II.

Bien entendu d'autres procédés de préparation peuvent également convenir.

Dans le cas où le groupement $X_2$ correspond à $QR_6$, et $X_1$ est différent de Hal, (composés de formule III), un procédé de préparation consiste à faire réagir un composé de formule II avec un nucléophile hétéroatomique de formule $R_6$-Q-E, dans laquelle $R_6$ et Q ont la même signification que dans la formule I et E est un cation, par exemple un métal alcalin ou alcalino terreux ou un ammonium quaternaire. La réaction est effectuée dans un solvant approprié en présence d'une base et éventuellement d'un catalyseur de transfert de phase et à des températures de réaction comprises généralement entre -30 °C et le reflux du solvant utilisé et dans un rapport molaire II : $R_6$ Q E compris de préférence entre 1,2 et 0,1.

Un autre procédé consiste à faire réagir le composé IIg dans les mêmes conditions avec le même nucléophile hétéroatomique pour obtenir un composé IIIa auquel on fixe ensuite le noyau triazole ou imidazole comme décrit précédemment.

Il est également possible d'atteindre les composés dans la formule desquels $R_6$ est un groupement alkyle inferieur éventuellement mono- ou polyhalogéné, en faisant réagir les composés III ou IIIa dans la formule desquels $R_6$ correspond à l'atome d'hydrogène avec un halogénure d'alkyle (éventuellement mono- ou polyhalogéné) dans un rapport molaire III ou IIIa : halogénure de préférence compris entre 1,1 et 0,2, en présence d'une base organique ou minérale. Si l'on est parti du composé IIIa on fixe ensuite un noyau triazole ou imidazole.

Dans le cas où $X_2$ correspond à un groupe hydroxyle (composés de formule III dans laquelle Q correspond à l'atome d'oxygène et $R_6$ à l'atome d'hydrogène) on peut également faire réagir en présence éventuellement d'un solvant organique inerte à une température comprise entre -30° et le reflux du solvant des composés de formule IIg ou II avec un hydroperoxyde, un hydroxyde, un oxyde ou un supéroxyde alcalin, alcalino-terreux ou métallique, dans un rapport molaire de préférence compris entre 1,1 et 0,2, puis fixer, dans le cas ou le composé IIg est le composé de départ, un noyau imidazole ou triazole.

Dans le cas où $X_2$ correspond à $N_3$ on fait réagir les composés IIg ou II avec un azidure de métal alcalin ou à'ammonium dans un rapport molaire de préférence compris entre 1,1 et 0,2, de préférence dans un solvant aprotique, à une température comprise entre -30° et le reflux du solvant, puis on fait réagir, si on est parti du composé IIg, le composé azidure avec un noyau imidazole ou triazole.

Dans le cas où $X_2$ correspond à un groupe hydroxyle on peut également faire réagir les composé IIc ou IId avec un peroxyde afin d'obtenir les composés epoxydes de formule IIIb pour IIc, et IIIc pour le composé

IId. Ces époxydes peuvent ensuite être hydratés en triol respectivement IIId, IIIe de façon bien connue. On cyclise en présence d'un agent de déshydratation comme les acides chlorhydrique, bromhydrique, sulfurique ou sulfonique éventuellement dans un solvant protique ou aprotique. Ce qui conduit au composé IIIa dans lequel $QR_6$ correspond à OH auquel on fixe ensuite un noyau triazole ou imidazole, ou au composé III dans le cas du composé IIIe. Eventuellement, si nécessaire, la fonction alcool peut être protégée de manière connue en soi pour les composés IIId et IIIe.

On peut également faire réagir les composés de formule II ou IIg avec le carboxylate (acylique, cyclique, saturé, insaturé ou aromatique) d'un métal alcalin ou alcalino terreux (notamment le benzoate de sodium) en présence d'un catalyseur de transfert de phase éventuellement en présence d'un solvant inerte ou polaire aprotique, éventuellement en présence d'eau et à des températures comprises entre 0° et le reflux de solvant considéré.

Dans le cas où $X_1$ et $X_2$ correspondent ensemble à un atome d'oxygène doublement lié au tétrahydrofuranne (composés de formule IV où $X_1$ correspond à l'atome d'oxygène), un procédé de préparation consiste à opérer à partir d'un composé de formule IIIa ou III dans laquelle $QR_6$ correspond à hydroxyle et $X_1$ à l'hydrogène, avec un oxydant bien connu pour obtenir un composé IV ou IVa auquel on greffe un noyau imidazole ou triazole.

On prépare les gem halogénés ($X_1$ = $X_2$ = Hal) par action du pentachlorure de phosphore sur la cétone (composé de formule IV où $X_1$ correspond à l'atome d'oxygène), en présence éventuellement d'un solvant inerte aprotique et éventuellement d'un halogénure d'ammonium quaternaire de préférence à température ambiante.

Four atteindre la fonction thiocétone (composé de formule IV ou $X_1$ correspond à l'atome de soufre) on effectue une thionation de la fonction cétone en faisant réagir les composés IV ou IVa par exemple avec $H_2S$ ou $P_2S_5$ dans un rapport molaire de préférence compris entre 1,1 et 0,2 en présence d'un solvant organique inerte (pyridine ou hydrocarbure par exemple) à température compris entre 20° et le reflux.

Dans le cas où $X_1$ et $X_2$ correspondent simultanément à $QR_6$ (composé de formule V) on fait réagir les composés de formule (IV) ou (IVa) dans laquelle $X_1$ (ou $X_2$) correspond à un atome d'oxygène ou de soufre doublement lié au tétrahydrofuranne avec un composé de formule $HQR_6$ ou Q correspond à 0 ou S dans un rapport molaire compris de préférence entre 1,1 et 0,2 en présence d'un catalyseur acide et dans un solvant approprié, pouvant être l'alcool ou le thiol lui-même, ou un solvant inerte comme les hydrocarbures ou les alcools. Si on est parti du composé de formule IVa on obtient le composé de formule Va, on greffe ensuite sur le composé de formule Va un noyau imidazole ou triazole.

Dans le cas où $X_1$ et $X_2$ correspondent à Q-$R_7$-Q et $R_7$ formant un radical unique divalent tel que défini précédemment, on fait réagir les composés de formule (IV) ou (IVa) avec un composé HQ-$R_7$-QH dans les mêmes conditions que pour les monoalcools ou les monothiols. Si on est parti du composé IVa on obtient le composé Vb auquel on fixe un imidazole ou triazole dans les mêmes conditions que précédemment.

Dans le cas où $X_2$ correspond à N-$R_8R_9$, un procédé de préparation consiste à faire réagir un composé de formule II ou de formule IIg avec un amine de formule H-N $R_8R_9$ dans un rapport molaire compris entre 1,1 et 0,2, éventuellement en présence d'une autre base organique ou minérale. Ce qui conduit au composé de formule VI dans le cas où l' on est parti du composé de formule II et au composé VIa auquel on fixe ultérieurement un noyau imidazole ou triazole dans le cas où l'on est parti du composé de formule IIg.

Dans le cas où $X_1$ (ou $X_2$) correspond au groupement = N-$R_{10}$ (composé de formule VII), on fait réagir un composé de formule IV ou IVa avec une amine de formule $R_{10}$-$NH_2$ ou un de ses sels dans un rapport molaire de préférence compris entre 1,1 et 0,2 dans un solvant organique inerte. Dans le cas du composé IVa on obtient le composé VIIa auquel on adjoint ensuite un noyau imidazole ou triazole.

On peut également faire réagir le composé de formule VII dans lequel $R_{10}$ correspond à l'hydroxyle avec un agent alkylant de formule $R_{11}$ D dans laquelle $R_{11}$ est un alkyle inférieur éventuellement substitué et dans laquelle D correspond à un groupe sulfonium, ammonium, sulfonate ou sulfate en présence d'une base organique ou minérale afin d'obtenir l'oxime substituée sur l'oxygène.

L'invention a également pour objet les composés éventuellement utilisables à titre d'intermédiaires dans les procédés de préparation décrit ci-dessus et de formule II, IIc, IId, IIe, IIf, IIg, IIh, III, IIIa, IIIb, IIIc, IIId, IIIe, IV, IVa, V, Va, Vb, VI, VIa, VII, VIIa dans lesquelles $R_1$ à $R_6$, Y, Tr, Im, m, n, $X_1$, $X_2$, Q, Hal, Z ont l'une quelconque des significations indiquées dans la description ci-avant.

La présente invention concerne également l'utilisation des composés de formule I à titre de fongicides.

Les composés selon l'invention peuvent être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en particulier des céréales telles que le blé, l'orge, le seigle, l'avoine et leurs hybrides et aussi le riz et le maïs Les composés selon l'invention sont actifs en particulier contre

les champignons notamment de type basidiomycètes, ascomycètes, adelomycètes ou fungi-imperfecti comme Botrytis cinerea, Erysiphe graminis, Puccinia recondita, Piricularia oryzae, Cercospora beticola, Puccinia striiformis, Erysiphe cichoracearum, Fusarium oxysporum (melonis), Pyrenophora avenae, Septoria tritici, Venturia inaequalis, Whetzelinia sclerotiorum, Monilia laxa, Mycosphaerella fijiensis, Marssonina panettoniana, Alternaria solani, Aspergillus niger, Cercospora arachidicola, Cladosporium herbarum, Helminthosporium oryzae, Penicillium expansum, Pestalozzia sp, Phialophora cinerescens, Phoma betae, Phoma foveata, Phoma lingam, Ustilago maydis, Verticillium dahliae, Ascochyta pisi, Guignardia bidwellii, Corticium rolfsii, Phomopsis viticola, Sclerotinia sclerotiorum, Sclerotinia minor, Coryneum cardinale, Rhizoctonia solani.

Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporiodies, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés de l'invention sont spécialement intéressants par leur spectre large au niveau des maladies des céréales (oïdium, rouille, piétin-verse, helminthosporioses, septorioses et fusarioses). Ils présentent également un grand intérêt eu raison de leur activité sur la pourriture grise (Botrytis) et les cercosporioses, et, de ce fait, ils peuvent être appliqués sur des cultures aussi variées que la vigne, les cultures maraîchères et l'arboriculture et les cultures tropicales telles que l'arachide, le bananier, le caféier, la noix de pécan et d'autres.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :

- Pullularia comme l'espèce P. pullulans,
- Chaetomium comme l'espèce C. globosum,
- Aspergillus comme l'espèce Aspergillus niger,
- Coniophora comme l'espèce C. puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés selon l'invention utilisés seuls ou sous la forme de compositions telles que définies ci-dessus dans les traitements du bois, sont généralement mis en oeuvre avec des solvants organiques et peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole, les insecticides tels que les pyrethroïdes ou les organochlorés.

Ils présentent enfin une excellente sélectivité vis-à-vis des cultures.

Ils s'appliquent avantageusement à des doses de 0,005 à 5 kg/ha, et plus spécifiquement de 0,01 à 0,5 kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Par le terme "support" dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non

ionique. On peut citer par exemple des sels d'acides polyacryliques, des sels d'acides lignosulfoniques, des sels d'acides phénolsulfoniques ou naphtalènesulfoniques, des polgcondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Par exemple, en plus du solvant, les concentrés émulsionnables peuvent contenir quand c'est nécessaire, 2 à 20 % d'additifs appropriés comme les stabilisants, les agents tensio-actifs, les agents de pénétration, les inhibiteurs de corrosion, les colorants ou les adhésifs précedemment cités.

Les doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

A titre d'exemple, voici la composition de quelques concentrés :

Exemple F (formulation) 1

| | |
|---|---|
| - matière active | 400 g/l |
| - dodécylbenzène sulfonate alcalin | 24 g/l |
| - nonylphénol oxyéthylé à 10 molécules d'oxyde d'éthylène | 16 g/l |
| - cyclohexanone | 200 g/l |
| - solvant aromatique | q.s.p 1 litre |

Selon une autre formule de concentré émulsionnable, on utilise :

Exemple F 2:

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc..

A titre d'exemple, voici diverses compositions de poudres mouillables :

Exemple F 3 :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

Exemple F 4 :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

Exemple F 5 :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

Exemple F 6 :

| | |
|---|---|
| - matière active | 250 g |
| - lignosulfonate de calcium | 45 g |
| - mélange équipondéral de craie de Champagne et d'hydroxyéthylcellulose | 19 g |
| - dibutylnaphtalène sulfonate de sodium | 15 g |
| - silice | 195 g |
| - craie de Champagne | 195 g |
| - kaolin | 281 g |

Une autre composition de poudre à pulvériser à 25 % utilise les constituants suivants :

Exemple F 7 :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |
| - aluminosilicate de sodium | 543 g |
| - kieselguhr | 165 g |

Une autre composition de poudre à pulvériser à 10 % utilise les constituants suivants :

Exemple F 8 :

| | |
|---|---|
| - matière active | 100 g |
| - mélange de sels de sodium de sulfates d'acides gras saturés | 30 g |
| - produit de condensation d'acide naphtalène sulfonique et de formaldéhyde | 50 g |
| - kaolin | 820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 A 25 % de matière active et 0 à 10 % d'additifs comme des stabilisants, des agents de modification à libération lente, des liants et des solvants.

Selon un exemple de composition de granulé, on utilise les constituants suivants :

Exemple F 9 :

| | |
|---|---|
| - matière active | 50 g |
| - épichlorhydrine | 2,5 g |
| - éther de cétyle et de polyglycol | 2,5 g |
| - polyéthylène glycol | 35 g |
| - kaolin (granulométrie : 0,3 à 0,8 mm) | 910 g. |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

Les exemples I à VII illustrent des modes particuliers de préparation de composés selon l'invention ainsi que ces composés eux-mêmes. La nomenclature des composés a été indiquée selon les normes françaises mis à part le fait que la numérotation des substituants a été mise avant les substituants eux-mêmes.

Exemple I : Préparation du 1-[4-bromo (2-(2,4-dichlorophényl) tétrahydro 2-furanylméthyl] 1 H 1,2,4-triazole. Composés n° 1a, 1b et 1a + 1b

Etape a) Préparation du 1-chloro 2-(2,4-dichlorophényl) 4-pentene 2-ol:

Un dérivé organomagnésien est préparé par addition d'une solution de bromure d'allyle (110 ml) dans l'éther éthylique (700 ml) et le tétrahydrofuranne (200 ml) avec du magnésium (110 g) entre 15 et 20° C en trois heures. On chauffe à reflux 30 mn, décante et lave à l'éther le résidu.

On additionne à cette phase organique, à -30° C, une solution de trichloro-alpha, 2, 4 acétophénone (175 g) dans le tétrahydrofuranne (250 g), neutralise à l'acide acétique. On lave à l'eau sèche sur sulfate de sodium, concentre puis distille sous vide. Une huile incolore est obtenue (205 g). Point d'ébullition (3 $10^{-2}$ mmHg) = 140-142° C.

Etape b) Préparation du 1-[2-(2,4-dichlorophényl)2-hydroxy 4-pentènyl] 1 H 1,2,4-triazole

Un mélange de produit obtenu à l'étape a) (106 g), de triazole (55 g) et de carbonate de potassium (160 g) est chauffé pendant quatre heures à 120° dans le diméthylformamide (600 ml). Les insolubles sont filtrés, lavés au diméthylformamide et le mélange réactionnel est concentré sous vide. Le résidu, dissous dans le chlorure de méthylène, est lavé à l'eau puis concentré. Le produit est obtenu par cristellisation dans l'acétate d'éthyle après dilution à l'heptane. Un solide rose pâle (97 g) est isolé fondant à 101°.

Etape c) Préparation des composés n° 1a et n° 1b

Le composé obtenu à l'étape b (35 g) dans le chloroforme (200 ml) est traité à 0° par du brome. Après décoloration, le solvant est évaporé et le résidu redissous dans le méthanol. On ajoute ensuite une solution aqueuse de potasse jusqu'à obtention d'un pH basique. Après évaporation du milieu sous vide, le résidu est extrait à l'acétate d'éthyle lavé à l'eau et concentré. L'huile obtenue (40 g) est constituée d'un mélange de deux diastéréoisomères en proportions sensiblement égales. Par chromatographie sur silice on isole successivement l'isomère le moins polaire n° 1a : cristaux blancs fondant à 83°, puis l'isomère le plus polaire n° 1b : cristaux blancs fondant à 94°. Après recristallisation on obtient la fondant à 96° et 1b fondant à 104°. Le mélange 50/50 de 1a et 1b fond à 74°.

De la même façon ont été préparés les composés suivants à partir des matières premières appropriées : 1-[4-bromo 2-(4-chlorophényl) tétrahydro 2-furanylméthyl] 1H 1,2,4-triazole 1C F = 74° Id F = 78° Ic+d F = 69°.

Exemple II - Préparation de 1-(2-(2,4-dichlorophényl) 4-hydroxy tétrahydro 2-furanylméthyl) 1 H 1,2,4-triazole. Composés n° 2a et n° 2b.

10 g de l'isomère la de l'exemple I, qui est l'isomère le moins polaire, dissous dans 30 ml de chlorobenzène, est chauffé à reflux pendant 48 h en présence de 20 g de benzoate de sodium, dans 30 ml d'eau, et de 1 g de catalyseur de transfert de phase "ADOGEN 464" methyltrialkylammonium chlorure. On obtient alors intermédiairement le composé 4-benzoate, c'est à dire le composé 4a ($X_1$ = H et

$X_2$ = 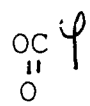 )

de de point de fusion 98° C ou le composé

4b ($X_1$ = 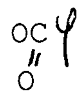

et $X_2$ = H) de point de fusion 140° C.

Après dilution à l'éther la phase organique est lavée à l'eau et réduite sous vide. Le résidu est alors traité à reflux pendant 3 heures dans le méthanol (100ml) en présence de potasse (7g). On refroidit, dilue à l'eau, extrait à l'acétate d'éthyle, lave à neutralité et purifie par chromatographie le produit brut obtenu après concentration sous vide. L'alcool 2a est isolé sous la forme d'une poudre blanche (2,8g) fondant à 193°.

En procédant de la même façon à partir de l'isomère le plus polaire 1b obtenu selon l'exemple I, on obtient l'alcool optiquement actif 2b qui se présente sous la forme d'une poudre blanche fondant à 162° C. Le mélange 50/50 2a et 2b est une huile.

Exemple III - Préparation de 1-(2-(2,4-dichlorophényl) 4-éthoxy tétrahydro 2-furanylméthyl) 1H 1, 2, 4-triazole. Composés n°3a et n° 3b.

L'alcool 2a (2,2 g) en solution dans le diméthylsulfoxyde (12ml) est traité successivement par de l'hydrure de sodium à 80 % (0,42 g) puis de l'iodure d'éthyle (1,15 ml). Après 15 minutes le milieu est dilué à l'eau et extrait à l'acétate d'éthyle. Après lavage à l'eau, le solvant est évaporé et le résidu purifié par chromatographie sur silice pour donner une huile incolore qui est l'isomère 3a qui cristallise par triturage au pentane fondant à 90° ; de même à partir de 2b on obtient l'isomère 3b poudre blanche fondant à 63°. Le mélange 50/50 de 3a + 3b est une huile. De la même façon ont été obtenus les composés de formule VIII réunis dans le tableau suivant en utilisant les alcools appropriés :

| n° | $X_1$ | $X_2$ | Point de fusion |
|---|---|---|---|
| 5a | H | O-$CH_3$ | huile |
| 5b | 0-$CH_3$ | H | huile |
| 6a | H | O-n-Pr | huile |
| 6b | O-n-Pr | H | huile |

Exemple IV - Préparation de 1-[2(2,4-dichlorophényl) 4-éthylthio tétrahydro 2-furanylméthyl] 1H 1,2,4-triazole. Composés n°7a et n°7b

Le bromure la (3,8 g) en solution dans le diméthylsulfoxyde (38 ml) contenant de l'eau (2 ml) est traité par de l'hydrogénosulfure de sodium (2,8 g) pendant 2 heures. De la potasse en poudre (3,3 g) est alors a joutée suivie d'iodure d'éthyle (4 ml). Après 10 minutes d'agitation, le milieu est dilué à l'eau et extrait à l'éther. Après sèchage et évaporation on obtient l'isomère 7a qui est une huile jaune (3,9 g) fondant à 88°.

En procédant de la même manière à partir de 1b on obtient 7b sous forme d'une poudre jaune pâle fondant à 64°. Le mélange des isomères est une huile. De la même façon ont été obtenus les composés de formule VIII réunis dans le tableau suivant en utilisant les thiols appropriés :

| n° | $X_1$ | $X_2$ | Point de fusion |
|---|---|---|---|
| 8a | H | S-$CH_3$ | 96 |
| 8b | S-$CH_3$ | H | huile |
| 9a | H | S-n-Pr | 110 |
| 9b | S-n-Pr | H | huile |
| 10a | H | S-2-ClEt | 127 |
| 10b | S-2-ClEt | H | huile |
| 11a | H | S-i-Bu | 68 |
| 11b | S-i-BU | H | huile |
| 12a | H | S-i-Pr | 75 |
| 12b | S-i-Pr | H | 65 |

Exemple V - Préparation de 1-[7-(2,4-dichloro phényl) 1,4,6-trioxo spiro [4,4] 7-nonanyl méthyl] 1 H 1,2,4-triazole . Composé n° 13

Etape a) Préparation du 1-chloro 2-(2,4-dichlorophényl) 3,4,5-pentanetriol.

La chlorhydrine obtenue à l'étape a) de l'exemple I (91 g) est époxydée dans le dichloro-1,2 éthane (125 ml) en présence d'acétylacétonate de vanadyl (5 g) et de péroxyde de tertiobutyle à 70 % (200 ml) par chauffage à reflux pendant 48 heures. Le milieu refroidi est dilué à l'eau, lavé plusieurs fois avec une solution de bisulfite de sodium puis concentré. Le résidu est alors converti en triol par chauffage dans l'eau (200 ml) et le dioxanne (200 ml) en présence d'acide perchlorique (5 ml) pendant 3 heures. Après dilution à l'eau, le milieu est extrait au toluène (300 ml) puis concentré.

Etape b) Préparation de la 2-(2,4-dichlorophényl) 2-chlorométhyl tétrahydro 4-furanone

Le résidu huileux obtenu à l'étape a) est alors chauffé dans le toluène (100 ml) et le butanol (200 ml) en présence d'acide paratoluènesulfonique (0,5 g) avec séparation de l'eau formée. Après évaporation du milieu réactionnel, le résidu est chromatographié sur silice (éluant acétate d'éthyle/heptane 40 : 60) pour donner une huile incolore (14,5 g) correspondant à un mélange de diastéréoisomères d'alcool qui est le 2-(2,4-dichlorophényl) 4-hydroxy 2-chlorométhyl tétrahydrofuranne. Ce produit est oxydé directement par l'anhydride chromique dans l'acide acétique pour donner après purification par chromatographie sur silice la furanone sous forme de cristaux blancs fondant à 99° C.

Etape c) Préparation de 7-chlorométhyl 7-(2,4-dichlorophényl) 1,4,6-trioxa spiro [4,4] nonane.

La furanone obtenue à l'étape b) (4,2 g), dans le toluène (50 ml) est chauffée à reflux en présence d'éthylène glycol (6,5 ml) et d'acide ptoluène sulfonique (0,1 g) avec séparation de l'eau formée jusqu'à disparition du produit de départ.

Le milieu est lavé à la soude puis diluée à l'eau, extrait à l'éther et concentré. On obtient un solide blanc (5,1 g) fondant à 99°.

Etepe d) Préparation du composé 13

L'halogénure de l'étape c) (5 g) dans le diméthylsulfoxyde (20 ml) est chauffé A 170° en présence de triazolylsodium (2,75 g) pendant 6 heures. Le milieu est versé dans l'eau, extrait à l'acétate d'éthyle, concentré et purifié par chromatographie sur silice. On isole des cristaux jaune pâle (3,6 g) qui fondent à 123° après recristallisation dans un mélange acétate d'éthyle/heptane.

Exemple VI - Préparation du 1-[4-chloro (2-(2,4-dichlorophényl) tétrehydro 2-furanylméthyl] 1 H 1,2,4-triazole. Composés n° 14e et n° 14b

Etepe a) Préparation du 2-(2,4-dichlorophényl) 1,4,5-trichloro 2-pentanol.

Le chlorhydrine obtenue à l'étape a) de l'exemple I en solution dans le dichlorométhane (150 ml) est traité par du chlore gazeux (13,4 g) à -15°. Le milieu est alors traité avec une solution de bisulfite de

sodium à 37 % (15 ml) lavé à l'au et séché puis évaporé. On obtient un produit brut sous forme d'une huile incolore (49,7 g) contenant environ 70 % du produit désiré sous forme d'un mélange de deux diastéréoisomères.

Etape b) Préparation du 1-(2,4-dichlorophényl) 1-(2,3-dichloro 1-propanyl) oxiranne

Une première méthode consiste à mettre la chlorhydrine brute (10,3 g) obtenue à l'étape a) ci-dessus en solution dans le méthanol (30 ml) et à la traiter par une solution de potasse méthanolique titrant 2,55 - $10^{-3}$ moles/1 (12 ml) à température ambiante. Le précipité est filtré et la solution méthanolique évaporée sous vide. Le résidu est purifié par chromatographie sur silice. Une huile incolore est obtenue (7,4 g).

Une seconde méthode consiste à mettre la chlorhydrine (19,9 g) obtenue à l'étape a de l'exemple I en solution dans le méthanol (75 ml) et à la traiter par une solution de potasse (4,9 g) dans le méthanol (20 ml) à température ambiante. Après filtration des insolubles et évaporation, on obtient l'époxyde (17,1 g) sous forme d'une huile jaune. Cet époxyde est traité par du chlore jusqu'à persistance de la couleur jaune (10,1 g) à -15°C. Le milieu est ensuite lavé avec une solution de bisulfite de sodium, de l'eau puis évaporé sous vide. Une huile jaune (20,8 g) constituée d'un mélange de deux diastéréoisomères dans un rapport 45/55 est obtenue.

Etape c) Préparation de 1-[4-chloro 2-(2,4-dichlorophényl) tétrahydro 2-furanylméthyl] 1 H 1,2,4-triazole

L'époxyde obtenu à l'étape b) (61,7 g) dans le butanol-1 (0,5 l) est chauffé à 90° pendant 6 heures en présence de triazolylsodium (18,6 g). Le précipité minéral est filtré et le butanol évaporé. Le résidu est purifié par chromatographie sur silice (éluant 48 % Acétate d'éthyle 48 % heptane 4 % méthanol) pour donner successivement le premier diastéréoisomère 14a fondant à 113° puis le 2ème diastéréoisomère 14b fondant à 97°. Le mélange 50/50 14a + 14b fond à 90°.

Exemple VII

Préparation de 1-[4-oxo 2-(2,4-dichlorophényl) tétrahydro 2-furanylméthyl] 1H 1,2,4 triazole composé 15

L'alcool 2a (37,7 g) est ajouté à -60° à une solution de diméthylsulfoxyde (17 ml), dans le dichlorométhane (120 ml), traité à -60° par une solution d'anhydride trifluoroacétique (25,4 ml) dans le dichlorométhane (60 ml). Après une demi-heure à -60° on laisse revenir à température ambiante puis on ajoute de la triéthylamine (48 ml). Le milieu est versé dans l'eau, extrait au dichlorométhane et évaporé. Une poudre blanche fondant à 91° est isolée par cristallisation dans l'éther.

Exemple VIII

Préparation de 1-[4,4-dichloro 2-(2,4 dichlorophényl) tétrahydro 2-furanylméthyl] 1H 1,2,4-triazole composé n° 20

Un mélange de cétone 15 (3,1 g), de pentachlorure de phosphore (2,3g), de dichlorométhane (30 ml) contenant du chlorure de triéthylbenzylammonium (0,25 g) est agité pendant 2 heures à température ambiante jusqu'à disparition du produit de départ. Le milieu est évaporé, dilué avec de l'eau (100 ml), neutralisé au bicarbonate de sodium et extrait à l'éther. Après séchage et évaporation, le résidu huileux est recristallisé dans l'éther isopropylique (2 fois). On obtient une poudre blanche (0,6 g) fondant à 138°.

Exemple IX - Préparation de 1-[4-diméthylamino 2-(2,4-dichlorophényl) tétrahydro 2-furanylméthyl] 1 H 1,2,4-triazole composé 16

A une solution de potasse (0,24 g) et de chlorhydrate de diméthylamine (1,05 g) dans le méthanol on ajoute successivement la cétone de l'exemple III, puis du cyanoborohydrure de sodium (0,24 g). Après 15 heures, le milieu est dilué à l'eau, extrait à l'éther. Le produit désiré est alors extrait de la phase organique par de l'acide chlorhydrique 6N (3 x 20 ml). Après neutralisation, extraction et chromatographie sur silice on isole 5 (1,6 g) sous forme d'une huile jaune pâle (mélange 50/50 de 2 isomères).

Exemple X - Préparation de 1-[4-méthoximino 2-(2,4-dichlorophényl) tétrahydro 2-furanylméthyl] 1H 1,2,4-triazole composé 17.

La cétone de l'exemple III (2g) dans l'éthanol (30 ml) est chauffée à reflux en présence de chlorhydrate de méthoxylamine (5,8 ml d'une solution à 25 % dans l'eau) pendant 2 heures. Le milieu est dilué à l'eau, extrait au dichlorométhane et évaporé. Le produit est isolé par cristallisation dans le mélange éther diisopropylique et heptane sous forme d'une poudre blanche fondant à 108° (mélange de deux isomères géométriques).

De la même façon ont été préparés les composés où

$R_{11}$ = H (4-hydroxymino) F = 195° 18

$R_{11}$ = 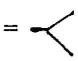 (4-isopropoxyimino) huile 19.

Les exemples XI et suivants illustrent les applications fongicides des composés selon l'invention.

dans ces exemples, les pulvérisations de solutions ou suspensions de matières actives sont effectuées dans des conditions telles que la pulvérisation d'une solution ou suspension de concentration égale à 1 g/l correspond en moyenne à l'application d'environ 2 microgrammes de matière active par $cm^2$ de feuille de la plante.

Dans les conditions des exemples XI et suivants, les composés illustrés n'ont pas présenté de phytotoxicité.

Dans ces exemples on considère qu'un produit exerce une protection totale vis à vis d'une maladie fongique lorsque la protection est à'au moins 95 % ; la protection est considérée comme bonne lorsqu'elle est d'au moins 80 % <mais inférieure à 95 %), comme assez bonne lorsqu'elle est d'au moins 70 % (mais inférieure à 80 %), comme moyenne lorsqu'elle est d'au moins 50 % (mais inférieure à 70 %).

Dans le présent expose les pourcentages sont, sauf indication contraire et sauf ceux concernant les rendements, des pourcentages pondéraux. Dons le cas où les pourcentages sont exprimés par rapport à la stoechiométrie, il s'agit de pourcentages molaires. En ce qui concerne les concentrations, certaines d'entre elles sont exprimées en ppm (partie par million) ce qui correspond à des mg/l.

Exemple XI - Test in vivo sur Botrytis cinerea sur tomate

On prépare par broyage fin une émulsion aqueuse de la matière active à tester ayant la composition suivante :

- matière active à tester : 60 mg
- Tween 80 (agent tensio actif) constitué d'un oléate de dérivé polyéxyéthylèné du sorbitan) dilué à 10 % dans l'eau : 0,3 ml
- on complète à 60 ml d'eau.

Cette émulsion aqueuse est ensuite diluée par de l'eau pour obtenir la concentration désirée.

Des tomates cultivées en serre (variété Marmande) agées de 30 à 40 jours sont traitées par pulvérisation avec des émulsions aqueuses (appelées bouillies) telles que définies ci-dessus et à diverses concentrations du composé à tester. L'essai est répété deux fois avec chaque concentration.

Après 24 ou 48 heures, les feuilles sont coupées et mises dans 2 boites de Pétri (diamètre 14 cm) dont le fond a été préalablement garni d'un disque de papier filtre humide (5 folioles par boite).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dons une solution nucritive (100.000 unités/$cm^3$).

Le contrôle est fait à 3 et 6 jours après la contamination par comparaison avec un témoin non traité.

Dans ces conditions on observe, à la dose de 1 g/l, une protection bonne ou totale avec les composés 1a, 1b, 1a + 1b, 3b, 5b, 6b, 8b, 9b, 12b, 14a, 14b, 14a + 14b, 17.

Exemple XII - Test in vivo sur Erysiphe graminis sur orge (oïdium de l'orge)

De l'orge, en godets, semée dans de la terre franche, est traitée au stade 10 cm de hauteur par pulvérisation d'une émulsion aqueuse (appelée bouillie) de concentration indiquée ci-après. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 14 jours après la contamination.

Dans ces conditions, on observe les résultats suivants : à la dose de 1 g/l, protection bonne ou totale avec les composés 1a, 1b, 1a + 1b, 2a, 2b, 3a, 3b, 3a + 3b, 4a, 4b, 5a, 5b, 6a, 6b, 7a + 7b, 8a, 8b, 13,

14a, 14b, 14a + 14b, 15, 17, 19, 20.

Exemple XIII - Test in vivo sur "Puccina recondita" responsable de le rouille du blé

Du blé, en godets, semé dans de la terre franche, est traité au stade 10 cm de hauteur par pulvérisetion avec des émulsions aqueuses (appelées bouillies) de même composition que celle décrite à l'exemple VIII et à diverses concentrations du composé à tester. L'essai est répété deux fois avec chaque concentration.

Au bout de 24 heures, une suspension aqueuse de spores (50000 sp/$cm^3$) est pulvérisée sur le blé ; cette suspension a été obtenue à partir de plante contaminés. On place ensuite le blé pendant 48 heures en cellule d'incubation à environ 18° C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

A la dose de 1 g/l, protection bonne ou totale avec les composés 1a, 1b, 1a + 1b, 3a, 3b, 3a + 3b, 5b, 6b, 7a, 7a + 7b, 8b, 12b, 13, 14a, 14b, 14a + 14b, 17, 19, 20.

Exemple XIV - Test in vivo sur "piricularia oryzae" responsable de la Piriculariose du riz (Rice Blast)

Du riz, en godets, semé dans un mélange 50/50 de tourbe enrichie et de pouzzolane, est traité au stade 10 cm de hauteur par pulvérisation d'une émulsion aqueuse (appelée bouillie) ci-dessus définie de concentration indiquée ci-après. L'essai est répété deux fois. Au bout de 48 heures, on traite par application sur les feuilles, par une suspension de spores obtenues en culture pure.

La lecture se fait 8 jours après la contamination. Dans ces conditions, on observe les résultats suivants : à la dose de 1 g/l, protection bonne ou totale avec les composés 1a, 1b, 1a + 1b, 3a, 3b, 6a, 8a, 8b, 9a, 9b, 10b, 11a, 11b, 12a, 12b, 14a, 14b, 14a + 14b, 15, 20.

Exemple XV - Test in vitro sur champignons des semences et champignons du sol

On étudie l'action des composés selon l'invention sur les champignons suivants responsables des maladies des céréales et autres végétaux :

1) Pyrenophorae avenae
2) Septoria nodorum
3) Helminthosporium teres
4) Fusarium roseum
5) Fusarium nivale
6) Fusarium culmorum
7) Rhizoctonia cerealis
8) Septoria tritici
9) Botrytis cinerea sensible au carbendazime et aux imides cycliques
10) Botrytis cinerea résistant au carbendazime et aux imides cycliques
11) Pseudocercosporella herpotrichoïdes
12) Fusarium oxysporum F.sp melonis
13) Rhizoctonia solani
14) Helminthosporium gramineum

Les chiffres figurant avant les noms seront utilisés pour représenter ces champignons dans le tableau (II).

Pour chaque essai, on opère de la manière suivante : un milieu nutritif constitué de pomme de terre, de glucose et de gelose (milieu PDA) est introduit en surfusion dans une série de boîtes de Pétri (20 ml par boîte) après stérilisation à l'autoclave à 120° C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après 24 ou 48 h chaque boîte est ensemencée par dépot d'un fragment de mycelium provenant d'une culture précédente du même champignon.

Les boîtes sont conservées pendant 2 à 10 jours (selon le champignon testé) à 22° C et on compare alors la croissance du champignon dans les boîtes contenant la matière active à tester, à celle du même champignon dans la boîte utilisée comme témoin.

On détermine ainsi pour chaque composé testé le taux d'inhibition du champignon considéré pour une

dose de 30 ppm. Les résultats sont indiqués dans le tableau ci-après.

| Composé | Champignons | | | | | | |
|---|---|---|---|---|---|---|---|
| n° | 1 | 2 | 3 | 4 | 5 | 6 | 7 |
| 1a | 100 | 100 | 100 | 95 | 100 | 100 | 95 |
| 1b | 100 | 90 | 100 | 100 | 100 | 100 | 90 |
| 1a + 1b | 100 | 95 | 100 | 95 | 100 | 100 | 90 |
| 2a | 50 | 95 | 80 | 50 | 80 | 100 | 80 |
| 2b | 80 | 110 | 80 | 50 | 90 | 90 | 80 |
| 3a | 95 | 90 | 100 | 50 | 80 | 80 | 90 |
| 3b | 95 | 95 | 100 | 80 | 90 | 100 | 90 |
| 3a + 3b | 80 | 0 | 80 | 0 | 80 | 80 | 80 |
| 4a | 90 | 0 | 90 | 80 | 80 | 90 | 90 |
| 4b | 95 | 80 | 95 | 80 | 80 | 100 | 90 |
| 5a | 50 | 90 | 50 | 50 | 50 | 50 | 50 |
| 5b | 100 | 100 | 100 | 90 | 95 | 100 | 95 |
| 6a | 50 | 90 | 50 | 50 | 50 | 50 | 50 |
| 6b | 100 | 95 | 95 | 80 | 90 | 90 | 90 |
| 7a | 95 | 95 | 95 | 0 | 0 | 0 | 50 |
| 7b | 95 | 90 | 95 | 0 | 0 | 0 | 50 |
| 7a + 7b | 50 | 95 | 50 | 0 | 0 | 0 | 50 |
| 8a | 95 | 80 | 95 | 80 | 50 | 100 | 80 |
| 8b | 100 | 90 | 95 | 80 | 80 | 90 | 80 |
| 9a | 95 | 90 | 95 | 80 | 80 | 90 | 80 |
| 9b | 80 | 95 | 95 | 0 | 0 | 80 | 80 |
| 10a | 80 | 0 | 80 | 50 | 90 | 95 | 80 |
| 10b | 90 | 80 | 90 | 0 | 0 | 50 | 80 |
| 11a | 90 | 50 | 95 | 0 | 0 | 0 | 80 |
| 11b | 90 | 80 | 95 | 0 | 0 | 0 | 80 |
| 12a | 90 | 80 | 95 | 0 | 80 | 80 | 80 |
| 12b | 95 | 90 | 95 | 80 | 80 | 90 | 80 |

| Composé n° | Champignons 1 | 2 | 3 | 4 | 5 | 6 | 7 |
|---|---|---|---|---|---|---|---|
| 13a | -- | 90 | -- | -- | -- | -- | -- |
| 14a | 95 | 95 | 100 | 95 | 100 | 100 | 90 |
| 14b | 95 | 90 | 100 | 90 | 95 | 100 | 90 |
| 14a+14b | 100 | 95 | 100 | 100 | 100 | 100 | 95 |
| 15 | 0 | 95 | 80 | 0 | 0 | 50 | 0 |
| 16 | 80 | 0 | 80 | 0 | 0 | 50 | 0 |
| 17 | 80 | 0 | 80 | 80 | 95 | 80 | 80 |
| 18 | | | | | | | |
| 19 | 90 | 0 | 80 | 80 | 90 | 50 | 80 |
| 20 | 90 | 80 | 95 | 90 | 95 | 90 | 90 |

| Composé n° | Champignons 8 | 9 | 10 | 11 | 12 | 13 | 14 |
|---|---|---|---|---|---|---|---|
| 1a | 100 | 100 | 100 | 100 | 100 | 80 | 100 |
| 1b | - | 95 | 95 | 100 | 100 | 80 | 100 |
| 1a + 1b | 100 | 95 | 95 | 100 | 100 | 90 | 95 |
| 2a | 0 | 95 | 95 | 100 | 0 | 0 | 80 |
| 2b | 0 | 80 | 50 | 100 | 80 | 0 | 80 |
| 3a | 95 | 90 | 90 | 95 | 0 | 0 | 90 |
| 3b | 100 | 95 | 100 | 95 | 80 | 80 | 95 |
| 3a + 3b | 0 | 80 | 95 | 0 | 0 | 0 | 80 |
| 4a | 0 | 95 | 95 | 0 | 0 | 0 | 90 |
| 4b | 0 | 95 | 95 | 90 | 95 | 0 | 95 |
| 5a | 0 | 0 | 0 | 100 | 0 | 0 | 50 |
| 5b | 0 | 95 | 90 | 100 | 95 | 90 | 90 |
| 6a | 0 | 50 | 50 | 100 | 0 | 0 | 50 |
| 6b | 50 | 95 | 95 | 100 | 80 | 80 | 95 |
| 7a | 0 | 95 | 95 | 95 | 0 | 80 | 80 |
| 7b | 0 | 90 | 95 | 90 | 0 | 50 | 50 |
| 7a + 7b | - | 95 | 95 | 100 | 50 | 80 | 50 |
| 8a | 0 | 80 | 80 | 80 | 80 | 80 | 95 |
| 8b | 0 | 95 | 95 | 90 | 80 | 80 | 95 |
| 9a | 0 | 90 | 90 | 90 | 80 | 80 | 95 |
| 9b | 0 | 95 | .90 | 100 | 80 | 80 | 50 |

| | | | | | | | |
|---|---|---|---|---|---|---|---|
| 10a | 50 | 80 | 0 | 0 | 0 | 0 | 0 |
| 10b | 0 | 90 | 95 | 80 | 50 | 50 | 90 |
| 11a | 0 | 0 | 80 | 50 | 50 | 50 | 95 |
| 11b | 0 | 80 | 80 | 80 | 80 | 80 | 95 |
| 12a | 0 | 50 | 80 | 80 | 0 | 80 | 90 |
| 12b | 0 | 95 | 95 | 90 | 80 | 80 | 100 |
| 13a | - | 0 | 0 | 100 | 50 | 0 | - |

| Composé | Champignons | | | | | | |
|---|---|---|---|---|---|---|---|
| n° | 8 | 9 | 10 | 11 | 12 | 13 | 14 |
| 14a | - | 100 | 100 | 100 | 100 | 80 | 100 |
| 14b | 0 | 95 | 95 | 100 | 80 | 80 | 100 |
| 14a+14b | 50 | 95 | 95 | 100 | 100 | 80 | 95 |
| 15 | - | 0 | 0 | 95 | 0 | 0 | 0 |
| 16 | 0 | 0 | 0 | 0 | 0 | 0 | 80 |
| 17 | 0 | 80 | 80 | 0 | 0 | 0 | 80 |
| 18 | | | | | | | |
| 19 | 0 | 0 | 0 | 0 | 0 | 0 | 90 |
| 20 | 0 | 80 | 90 | 100 | 50 | 80 | 90 |

EP0 151084

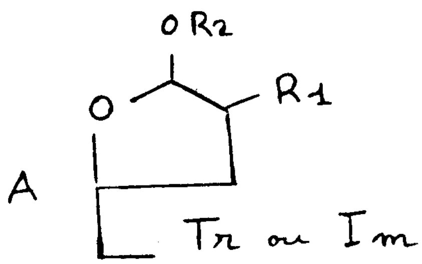

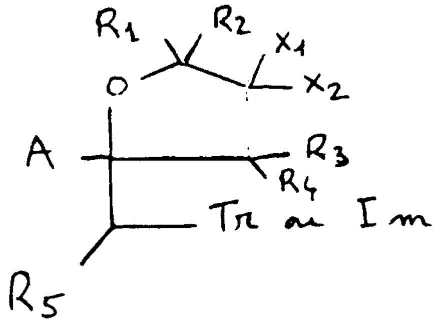

(I)

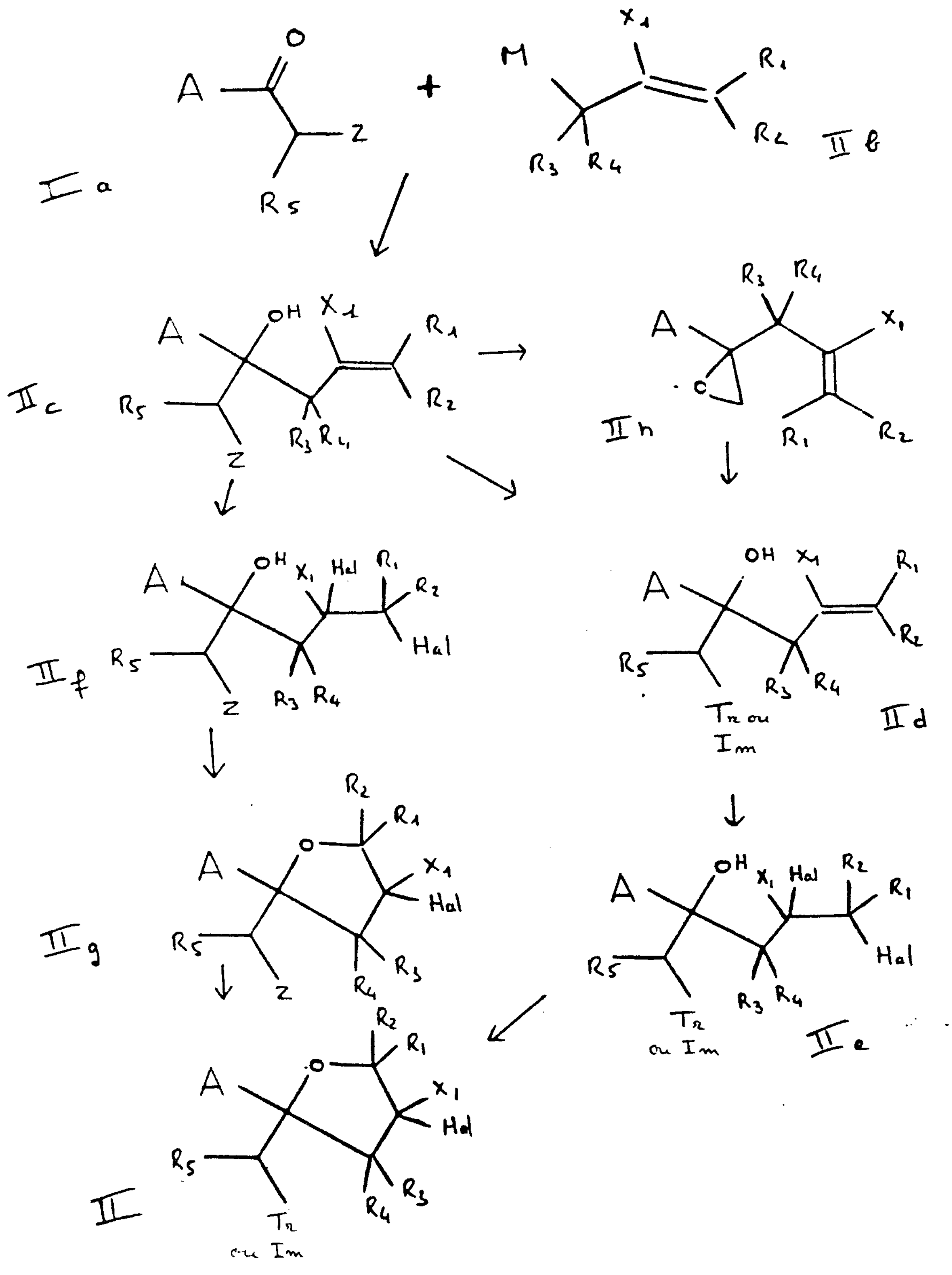

I a

II b

II c

II h

II f

II d

II g

II e

II

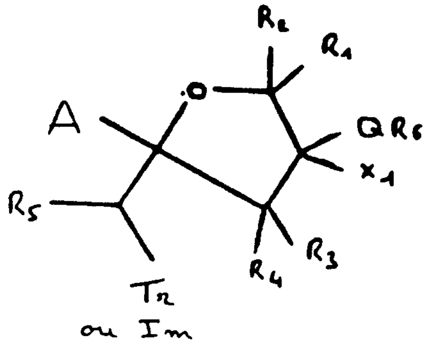

III

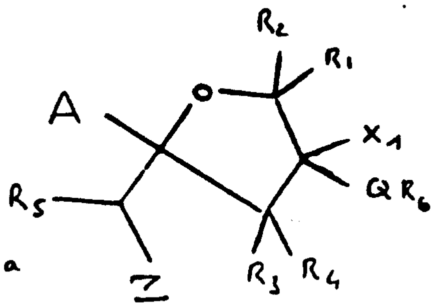

IIIa

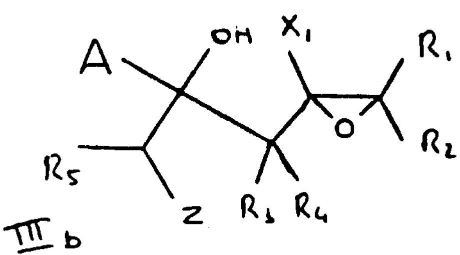

IIIb

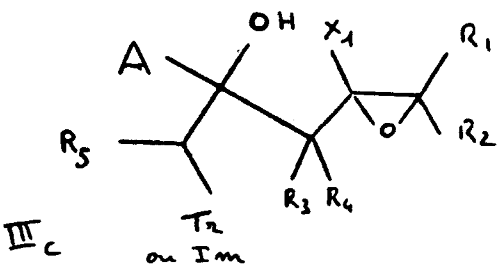

IIIc

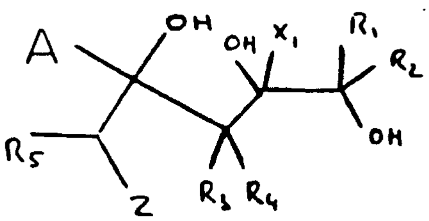

IIId

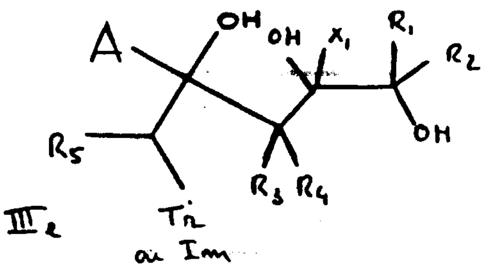

IIIe

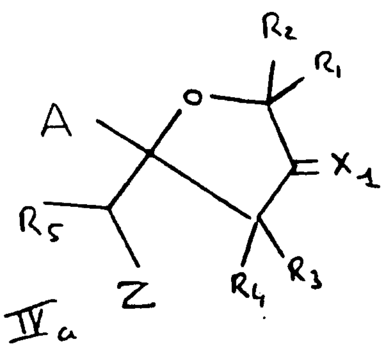

IVa

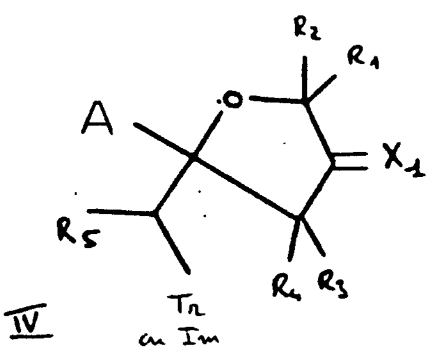

IV

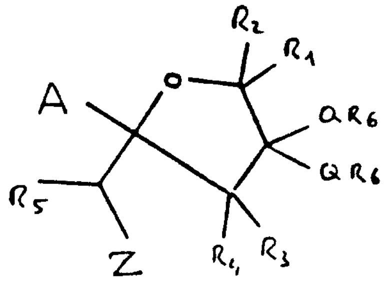

Va

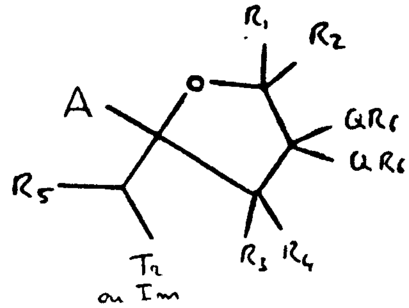

V

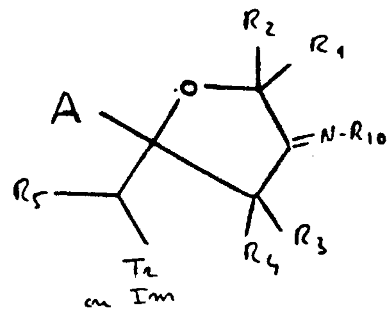

VII

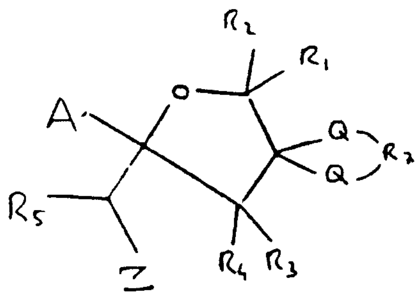

Vb

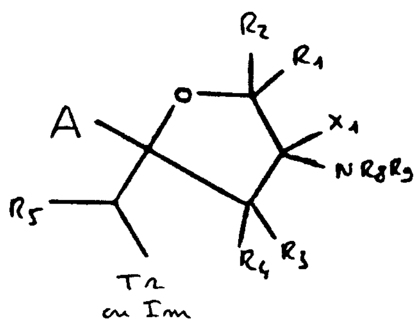

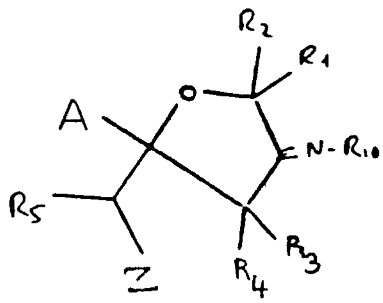

VIIa

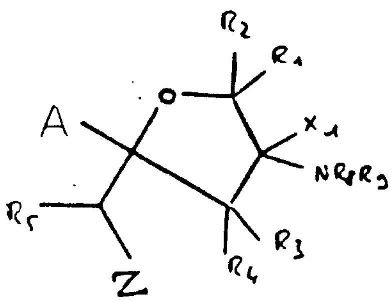

IIa

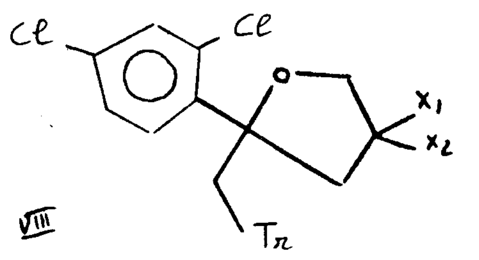

VIII

## Revendications

**1.** Composés à groupements triazole ou imidazole,tétrahydrofuranne et phényle caractérisés en ce qu'ils répondent à la formule générale (I)

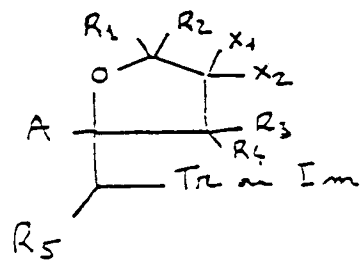

dans laquelle

A est le groupement $(Y)_n$-Ph-$(CH_2)$m- où Ph est un noyau phénylène et m = 0 ou 1,

Y est un atome d'halogène ou un groupe cyano ou nitro, ou un groupe alkyle ou alkoxy éventuellement halogéné,

n est un nombre entier positif ou nul, inférieur à 6,les groupements Y pouvant être identiques ou différents lorsque n est plus grand que 1,

Tr représente un groupe 1,2,4-triazole 1-yl et Im un groupe 1,3-imidiazole 1-yl,

$R_1$ à $R_5$, identiques ou différents, représentent l'atome d'hydrogène ou un radical alkyle comportant au plus six atomes de carbone,linéaire ou ramifié, cycloalkyle comportant au plus six atomes de carbone, phényle, benzyle, alkoxy comportant au plus six atomes de carbone, ces divers radicaux pouvant être éventuellement substitués, par un ou plusieurs atomes d'halogènes ou radicaux alkoxy comportant au plus six atomes de carbone;

$X_1$, $X_2$, identiques ou différents, représentent l'atome d'hydrogène, un atome d'halogène, un radical alkyle comportant au plus six atomes de carbone, un radical cycloalkyle comportant au plus six atomes de carbone, un radical phényle, ou benzyle, ces divers radicaux pouvant être éventuellement substitués de façon similaires aux groupes $R_1$ à $R_5$,

un groupement Q-$R_6$ dans lequel Q représente O ou S, et $R_6$ représente l'atome d'hydrogène ou un radical alkyle comportant au plus six atomes de carbone, un radical cycloalkyle comportant au plus six atomes de carbone, phényle, benzyle,benzoyle ou acyle, ces radicaux pouvant être substitués de façon similaire aux groupes $R_1$ à $R_5$ et pouvant avoir des significations identiques ou différentes lorsque $X_1$ et $X_2$ correspondent chacun à $OR_6$ ou $SR_6$,

un groupement de formule -Q-$R_7$-Q- où Q a la même signification que précédemment et dans laquelle $R_7$ est un radical divalent hydrocarboné,comprenant de 2 à 6 atomes de carbones, l'un de ces atomes de carbone pouvant être remplacé par un atome d'oxygène ou d'azote, ledit radical unique divalent étant lui-même éventuellement substitué par un ou plusieurs atomes d'halogène ou hydroxy, ou radicaux alkyle comportant au plus six atomes de carbone, alkoxy comportant au plus six atomes de carbone,

un groupement -N-$R_8R_9$ dans lequel $R_8$ et $R_9$, identiques ou différents, représentent l'atome d'hydrogène ou un radical alkyle comportant au plus six atomes de carbone, un radical cycloalkyle comportant au plus six atomes de carbone, phényle ces divers radicaux pouvant être éventuellement substitués de façon similaire aux groupes $R_1$ à $R_5$, ou bien $R_8$ et $R_9$ peuvent former ensemble un radical unique divalent hydrocarboné, comprenant de 2 à 6 atomes de carbones, l'un de ces atomes de carbone pouvant être remplacé par un atome d'oxygène, de soufre ou d'azote, ledit radical unique divalent étant lui-même éventuellement substitué par un ou plusieurs atomes d'halogène, groupes hydroxy ou radicaux alkyle comportant au plus six atomes de carbone ou radicaux alkoxy éventuellement halogénés comportant au plus six atomes de carbone;

un groupement = N-$R_{10}$ dans lequel $R_{10}$ correspond à un radical alkyle comportant au plus six atomes de carbone, un radical cycloalkyle comportant au plus six atomes de carbone, à un groupement de formule $OR_{11}$ dans laquelle $R_{11}$ est un alkyle, ces radicaux pouvant être substitués de façon similaire aux groupes $R_1$ à $R_5$, un groupement hydroxy,

un groupement $N_3$ à la condition que $X_2$ soit différent de $N_3$,

$X_1$ ou $X_2$ peuvent correspondre à = O ou = S,l'autre groupe étant supprimé,

aux conditions générales que $X_1$ et $X_2$ ne puissent pas correspondre simultanément à l'atome d'hydrogène, à un radical alkyle comportant au plus six atomes de carbone, un radical cycloalkyle comportant au plus six atomes de carbone, un radical phényle ou un radical benzyle,et si $X_1$ et $X_2$ sont différents et que l'un des deux est $OR_6$ alors, dans ce cas là seulement,$R_6$ ne peut être que le groupe benzoyle ou acyle.

et leurs formes salifiées.

2. Composés selon la revendication 1 caractérisés en ce que m = 0, n = 1, 2 ou 3 et Y est un atome d'halogène.

3. Composés selon les revendications 1 et 2 caractérisés en ce que n = 1, ou 2 et Y est un atome d'halogène placé en ortho et/ou en para.

4. Composés selon la revendication 3 caractérisés en ce que Y est un atome de chlore ou de brome.

5. Composés triazole selon l'une des revendications 1 à 4 caractérisés en ce qu'ils répondent à la formule II

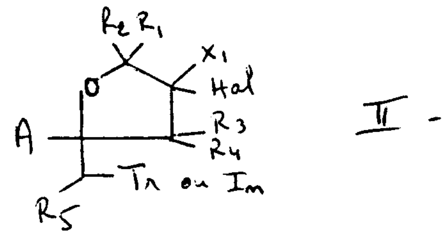

dans laquelle Y, m, n, ont l'une des significations indiquées dans le composé de formule I, $R_1$ à $R_5$ correspondent à un atome d'hydrogène, à un groupement alkyle ou cycloalkyle comportant au plus six atomes de carbone, Hal correspond à un atome d'halogène,et $X_1$ est un atome d'halogène ou d'hydrogène.

6. Composés selon la revendication 5 caractérisés en ce que Hal est le chlore ou le brome et $X_1$ est le chlore ou le brome.

7. Composés triazole selon l'une des revendications 1 à 4 caractérisés en ce qu'ils répondent à la formule III

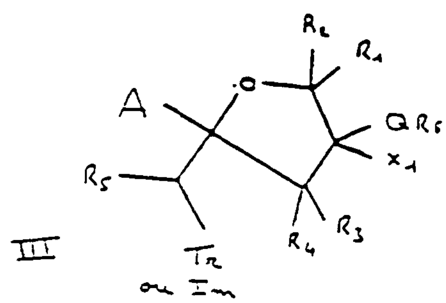

dans laquelle Y, n, m, ont l'une des significations indiquées dans le composé de formule I, $R_1$ à $R_5$ correspondent à un atome d'hydrogène, à un groupement alkyle ou cycloalkyle comportant au plus six atomes de carbone, $X_1$ correspond à un atome d'hydrogène, à un groupement alkyle ou cycloalkyle comportant au plus six atomes de carbone, Q a la même signification que dans la formule I, $R_6$ correspond à un atome d'hydrogène, à un groupement alkyle ou cycloalkyle comportant au plus six atomes de carbone.

8. Composés selon la revendication 7 caractérisés en ce que les groupements $R_1$ à $R_5$ correspondent à un atome d'hydrogène, à un groupement alkyle ou cycloalkyle comportant au plus six atomes de carbone et $R_6$ correspond à un groupe alkyle, cycloalkyle comportant au plus six atomes de carbone éventuellement substitué de façon similaire aux groupes R1 à R5.

9. Procédé de préparation des composés de formule II, dans laquelle $R_1$ à $R_5$, A, Y, n, $X_1$ ont la même

signification que dans la revendication 1 et Hal correspond à un atome d'halogène, caractérisé en ce qu'il consiste dans les étapes suivantes :

Etape a) On fait réagir une halogènocétone de formule IIa,

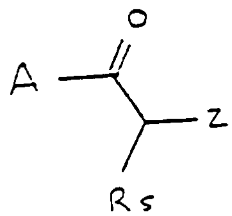

IIa

dans laquelle Y, n, $R_5$ ont la même signification que celle donnée pour les composés de formule I et Z correspond à un atome d'halogène, avec un organométallique de formule IIb,

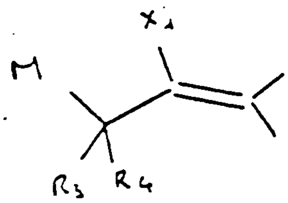

IIb

pour obtenir le composé de formule IIc,

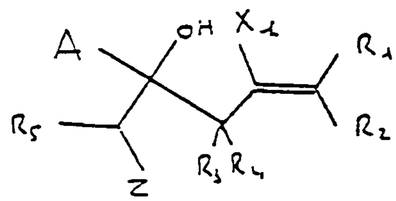

IIc

Etape b) On fait réagir ensuite le composé de formule IIc avec un imidazole ou un triazole non substitué en présence de base organique ou minérale et dans un solvant approprié,

Etape c) On additionne sur le composé IId

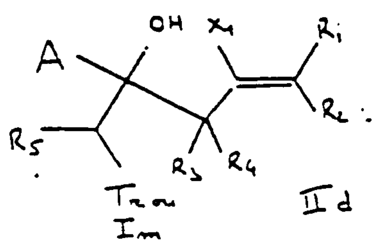

IId

une molécule d'halogène ou d'halogène mixte dans un solvant inerte,ce qui conduit au composé IIe,

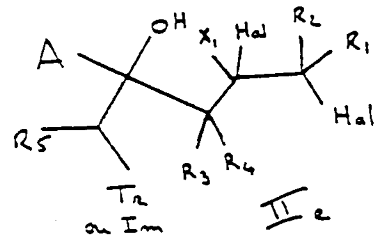

Etape d) Le composé II est obtenu par cyclisation du composé IIe en présence d'une base organique ou minérale.

**10.** Procédé de préparation des composés de formule II, dans laquelle $R_1$ à $R_5$, Y, n, A, $X_1$ ont la même signification que dans la revendication 1 et Hal correspond à un atome d'halogène, caractérisé en ce que on additionne sur le composé IIc selon la revendication 9 une molécule d'halogène ou d'halogène mixte dans un solvant inerte pour obtenir le composé IIf,

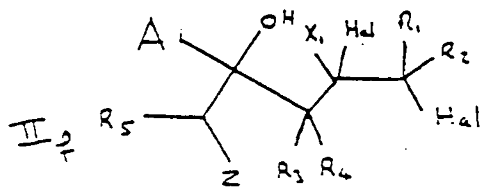

celui-ci étant cyclisé en présence d'une base organique ou minérale pour conduire au composé IIg,

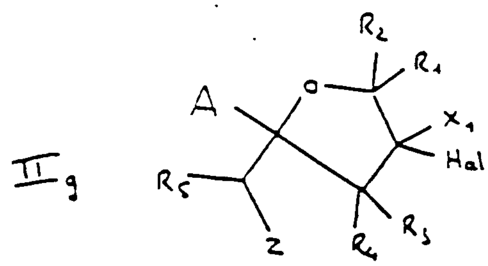

auquel on fixe un groupement triazole ou imidazole non substitué en présence de base organique ou minérale et dans un solvant approprié.

**11.** Procédé de préparation des composés de formules III ou V,

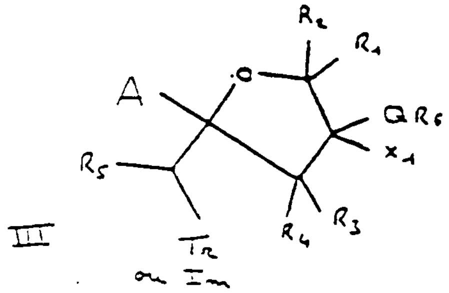

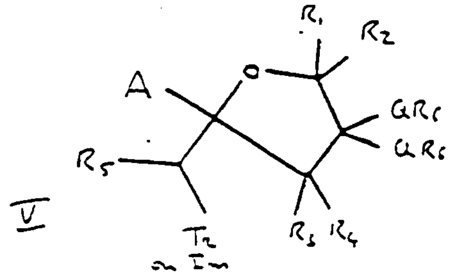

dans lesquelles $R_1$ à $R_6$, Y, n, A, $X_1$ ont la même signification que dans la revendication 1 et Q correspond à un atome d'oxygène ou de soufre, caractérisé en ce que on fait réagir un composé de formule II ou IIg avec un nucléophile hétéroatomique de formule $R_6$-Q-E dans laquelle $R_6$ et Q ont la même signification que dans le composé de formule I et E est un cation en présence d'une base et

éventuellement d'un catalyseur de transfert de phase et dans le cas où l'on est parti du composé IIg on fixe un imidazole ou triazole non substitué.

**12.** Procédé de préparation des composés de formule III dans laquelle $R_6$ est un groupement alkyle comportant au plus six atomes de carbone éventuellement mono- ou polyhalogéné, caractérisé en ce que l'on fait réagir un composé de formule III ou IIIa dans laquelle $R_6$ correspond à l'atome d'hydrogène avec un halogénure d'alkyle (éventuellement mono- ou polyhalogéné) en présence d'une base organique ou minérale et en ce que l'on fixe ensuite un noyau imidazole ou triazole si l'on est parti du composé IIIa

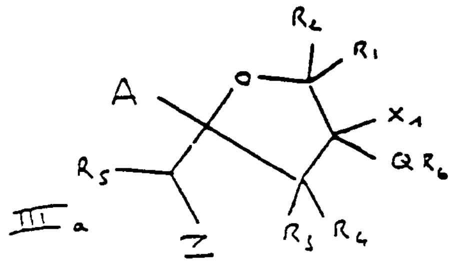

**13.** Procédé de préparation des composés de formule IV

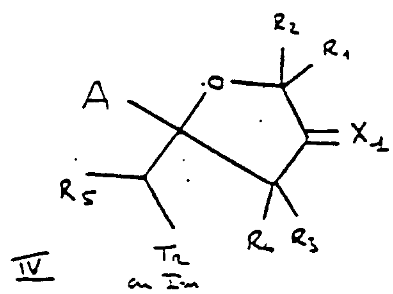

dans laquelle $R_1$ à $R_5$, n, Y, A, ont la même signification que dans la revendication 1 ou $X_1$ et $X_2$ ensemble correspondent à l'atome d'oxygène, caractérisé en ce qu'on oxyde un composé de formule III ou IIIa dans lesquelles Y, n, $R_1$ à $R_5$, ont la même signification que dans la revendication 1, Z est un atome d'halogène et dans laquelle $QR_6$ correspond à un groupement hydroxyle et $X_1$ à un atome d'hydrogène, et en ce que lorsque l'on est parti du composé IIIa on fixe ensuite un noyau imidazole ou triazole non substitué.

**14.** Procédé de préparation des composés de formule IV où $X_1$ et $X_2$ ensemble correspondent à l'atome de soufre, et Y, n, $R_1$ à $R_5$, ont la même signification que dans la revendication 1, caractérisé en ce que le composé de formule IV où $X_1$ et $X_2$ ensemble correspondent à l'atome d'oxygène, est soumis à une thionation.

**15.** Procédé de préparation des composés de formule V caractérisé en ce que on fait réagir les composés de formule IV ou IVa

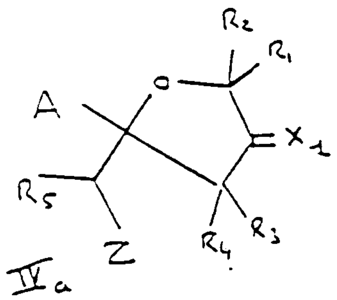

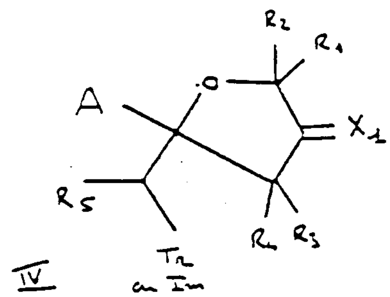

dans laquelle $X_1$ et $X_2$ ensemble correspondent à l'atome d'oxygène ou de soufre doublement lié au tétrahydrofuranne avec un composé de formule $HQR_6$ ou Q correspond à O ou S en présence d'un catalyseur acide et dans un solvant approprié, pouvant être l'alcool ou le thiol lui-même, ou un solvant inerte ; si on est parti du composé de formule IVa on obtient le composé de formule Va, on greffe ensuite sur le composé de formule Va un noyau imidazole ou triazole.

**16.** Procédé de préparation des composés de formule II dans laquelle $R_1$ à $R_5$, n, Y, A, ont la même signification que dans la revendication 1 ou $X_1$ et $X_2$ correspondent à l'atome d'halogène, de préférence le chlore ou le brome caractérisé en ce qu'on oxyde un composé de formule III ou IIIa dans lesquelles Y, n, $R_1$ à $R_5$, A, ont la même signification que dans la revendication 1, Z est un atome d'halogène et dans laquelle $QR_6$ correspond à un groupement hydroxyle et $X_1$ à un atome d'hydrogène puis en ce que on ajoute du pentachlorure de phosphore sur la cétone (composé de formule IV où $X_1$ correspond à l'atome d'oxygène), en présence éventuellement d'un solvant polaire aprotique et éventuellement d'un halogénure d'ammonium quaternaire de préférence à température ambiante.

**17.** Composés éventuellement utilisables à titre d'intermédiaires dans les procédés de préparation des revendications 9 à 16, caractérisés en ce qu'ils répondent aux formules IIIc et IIIe,

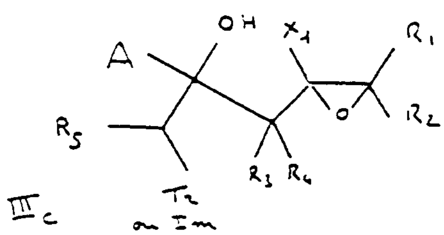

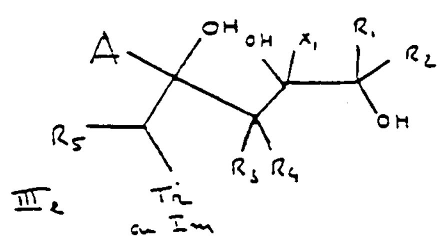

dans lesquelles $R_1$ à $R_5$, Y, A, m, n, $X_1$, $X_2$, ont l'une quelconque des significations indiquées à la revendication 1 ou 8.

**18.** Utilisation des composés selon l'une des revendications 1 à 8 à titre de fongicide, notamment sous forme de compositions fongicides contenant comme matière active un composé selon l'une des revendications 1 à 8,de préférence 0,5 à 95 % en poids, cette matière active étant en association avec au moins un support inerte, acceptable en agriculture.

**19.** Procédé pour lutter contre les maladies fongiques des cultures, caractérisé en ce qu'on applique une dose efficace d'une matière active selon l'une des revendications 1 à 8, de préférence à raison de 0,005 à 5 kg/ha, avantageusement de 0,01 à 0,5 kg/ha.

## Claims

**1.** Compounds containing triazole or imidazole, tetrahydrofuran and phenyl groups, characterised in that they correspond to the general formula (I)

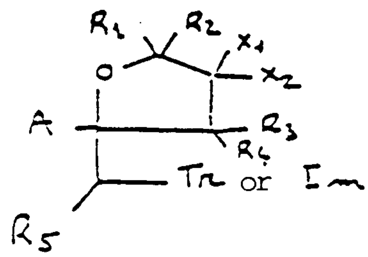

in which

A is a group $(Y)_n$-Ph-$(CH_2)_m$-, where Ph is a phenylene ring and m = 0 or 1,

Y is a halogen atom or a cyano or nitro group, or an optionally halogenated alkyl or alkoxy group,

n is 0 or a positive integer less than 6, it being possible for the groups Y to be identical or different when n is greater than 1,

Tr represents a 1,2,4-triazol-1-yl group and Im a 1,3-imidazol-1-yl group,

$R_1$ to $R_5$, which may be identical or different, represent a hydrogen atom or a linear or branched all radical containing at most six carbon atoms, a cycloalkyl radical containing at most six carbon atoms, a phenyl or benzyl radical or an alkoxy radical containing at most six carbon atoms, it being possible for these various radicals to be optionally substituted with one or more halogen atoms or alkoxy radicals containing at most six carbon atoms;

$X_1$ and $X_2$, which may be identical or different, represent a hydrogen atom, a halogen atom, an alkyl radical containing at most six carbon atoms, a cycloalkyl radical containing at most six carbon atoms or a phenyl or benzyl radical, it being possible for these various radicals to be optionally substituted in a manner similar to the groups $R_1$ to $R_5$,

a group Q-$R_6$, in which Q represents O or S, and $R_6$ represents a hydrogen atom or an alkyl radical containing at most six carbon atoms, a cycloalkyl radical containing at most six carbon atoms or a phenyl, benzyl, benzoyl or acyl radical, it being possible for these radicals to be substituted in a manner similar to the groups $R_1$ to $R_5$ and to have identical or different meanings when $X_1$ and $X_2$ each correspond to $OR_6$ or $SR_6$,

a group of formula -Q-$R_7$-Q-, where Q has the same meaning as above and in which $R_7$ is a divalent hydrocarbon radical comprising from 2 to 6 carbon atoms, it being possible for one of these carbon atoms to be replaced by an oxygen or nitrogen atom, the said single divalent radical itself being optionally substituted with one or more halogen atoms or hydroxyl groups, alkyl radicals containing at most six carbon atoms or alkoxy radicals containing at most six carbon atoms,

a group -N-$R_8R_9$, in which $R_8$ and $R_9$, which may be identical or different, represent a hydrogen atom or an alkyl radical containing at most six carbon atoms, a cycloalkyl radical containing at most six carbon atoms or a phenyl radical, it being possible for these various radicals to be optionally substituted in a manner similar to the groups $R_1$ to $R_5$, or alternatively $R_8$ and $R_9$ together can form a single divalent hydrocarbon radical comprising from 2 to 6 carbon atoms, it being possible for one of these carbon atoms to be replaced by an oxygen, sulphur or nitrogen atom, the said single divalent radical itself being optionally substituted with one or more halogen atoms, hydroxyl groups, alkyl radicals containing at most six carbon atoms or optionally halogenated alkoxy radicals containing at most six carbon atoms,

a group =N-$R_{10}$, in which $R_{10}$ corresponds to an alkyl radical containing at most six carbon atoms, or a cycloalkyl radical containing at most six carbon atoms, or to a group of formula $OR_{11}$ in which $R_{11}$ is an alkyl, it being possible for these radicals to be substituted in a manner similar to the groups $R_1$ to $R_5$, or a hydroxyl group,

a group $N_3$, on condition that $X_2$ is other than $N_3$,

$X_1$ or $X_2$ can correspond to =O or =S, the other group being eliminated,

on the general conditions that $X_1$ and $X_2$ cannot simultaneously correspond to a hydrogen atom or to an alkyl radical containing at most six carbon atoms, a cycloalkyl radical containing at most six carbon atoms, a phenyl radical or a benzyl radical, and if $X_1$ and $X_2$ are different and either one of them is $OR_6$, then, in this case only, $R_6$ can be only a benzoyl or acyl group;

and their salified forms.

**2.** Compounds according to Claim 1, characterised in that m = 0, n = 1, 2 or 3 and Y is a halogen atom.

**3.** Compounds according to Claims 1 and 2, characterised in that n = 1 or 2 and Y is a halogen atom in the ortho and/or para position.

**4.** Compounds according to Claim 3, characterised in that Y is a chlorine or bromine atom.

**5.** Triazole compounds according to one of Claims 1 to 4, characterised in that they correspond to the formula II

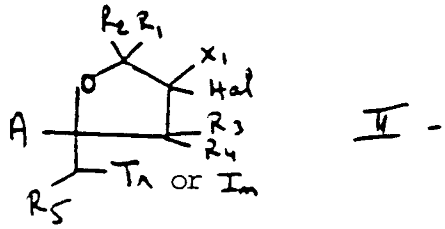

in which Y, m and n have one of the meanings indicated in the compound of formula I, $R_1$ to $R_5$ correspond to a hydrogen atom or to an alkyl or cycloalkyl group containing at most six carbon atoms, Hal corresponds to a halogen atom and $X_1$ is a halogen or hydrogen atom.

**6.** Compounds according to Claim 5, characterised in that Hal is chlorine or bromine and $X_1$ is chlorine or bromine.

**7.** Triazole compounds according to one of Claims 1 to 4, characterised in that they correspond to the formula III

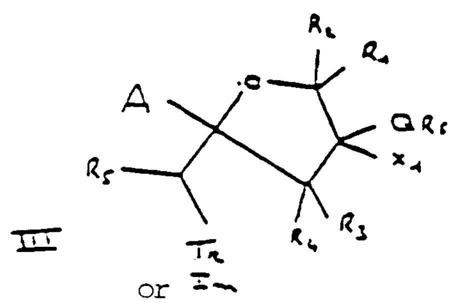

in which Y, n and m have one of the meanings indicated in the compound of the formula I, $R_1$ to $R_5$ correspond to a hydrogen atom or to an alkyl or cycloalkyl group containing at most six carbon atoms, $X_1$ corresponds to a hydrogen atom or to an alkyl or cycloalkyl group containing at most six carbon atoms, Q has the same meaning as in the formula I and $R_6$ corresponds to a hydrogen atom or to an alkyl or cycloalkyl group containing at most six carbon atoms.

**8.** Compounds according to Claim 7, characterised in that the groups $R_1$ to $R_5$ correspond to a hydrogen atom or to an alkyl or cycloalkyl group containing at most six carbon atoms, and $R_6$ corresponds to an alkyl or cycloalkyl group containing at most six carbon atoms and optionally substituted in a manner similar to the groups $R_1$ to $R_5$.

**9.** Process for the preparation of the compounds of formula II, in which $R_1$ to $R_5$, A, Y, n and $X_1$ has the same meaning as in Claim 1 and Hal corresponds to a halogen atom, characterised in that it consists of the following steps:
Step a) A halo ketone of formula IIa,

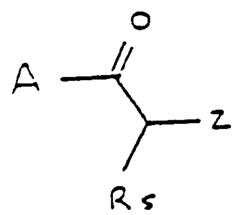

IIa

in which Y, n and $R_5$ have the same meaning as that given for the compounds of formula I and Z corresponds to a halogen atom, is reacted with an organometallic compound of formula IIb,

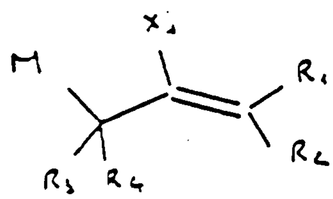

IIb

to obtain the compound of formula IIc,

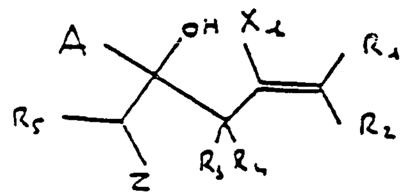

IIc

Step b) The compound of formula IIc is then reacted with an unsubstituted imidazole or triazole in the presence of an organic or inorganic base and in a suitable solvent,

Step c) A halogen molecule or mixed halogen molecule is added to the compound IId

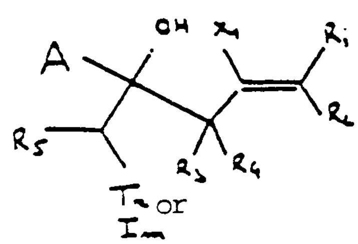

IId

in an inert solvent, leading to the compound IIe,

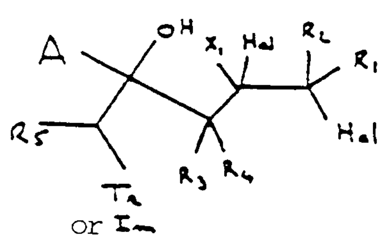

IIe

Step d) The compound II is obtained by cyclisation of the compound IIe in the presence of an organic or inorganic base.

**10.** Process for the preparation of the compounds of formula II, in which $R_1$ to $R_5$, Y, n, A and $X_1$ have the same meaning as in Claim 1 and Hal corresponds to a halogen atom, characterised in that a halogen molecule or mixed halogen molecule is added in an inert solvent to the compound IIc according to Claim 9, to obtain the compound IIf,

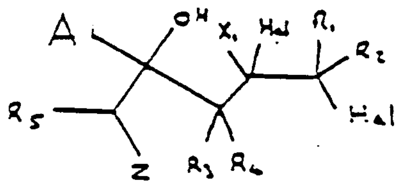

IIf

the latter being cyclised in the presence of an organic or inorganic base to lead to the compound IIg,

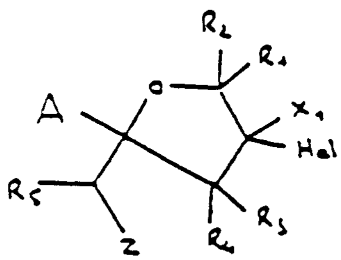

IIg

to which an unsubstituted triazole or imidazole group is attached in the presence of an organic or inorganic base and in a suitable solvent.

**11.** Process for the preparation of the compounds of formula III or V,

III 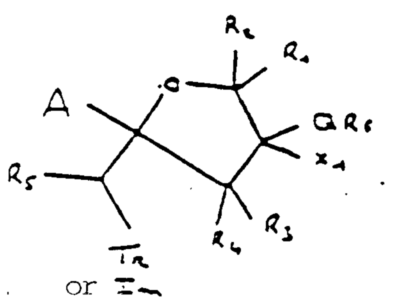

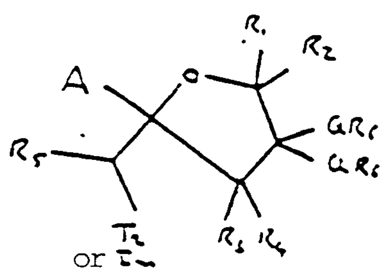

V

in which $R_1$ to $R_6$, Y, n, A and $X_1$ have the same meaning as in Claim 1 and Q corresponds to an oxygen or sulphur atom, characterised in that a compound of formula II or IIg is reacted with a nucleophile containing a hetero atom, of formula $R_6$-Q-E in which $R_6$ and Q have the same meaning as in the compound of formula I and E is a cation, in the presence of a base and optionally of a phase transfer catalyst, and in the case where the starting material is the compound IIg, an unsubstituted imidazole or triazole is attached.

**12.** Process for the preparation of the compounds of formula III in which $R_6$ is an optionally mono- or polyhalogenated alkyl group containing at most six carbon atoms, characterised in that a compound of formula III or IIIa in which $R_6$ corresponds to a hydrogen atom is reacted with an alkyl halide (where

appropriate mono- or polyhalogenated) in the presence of an organic or inorganic base, and in that an imidazole or triazole ring is then attached if the starting material is the compound IIIa

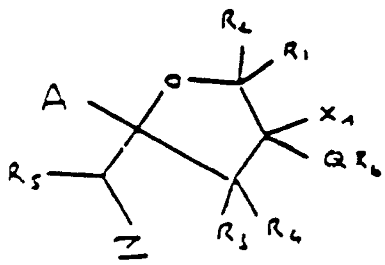

IIIa

**13.** Process for the preparation of the compounds of formula IV

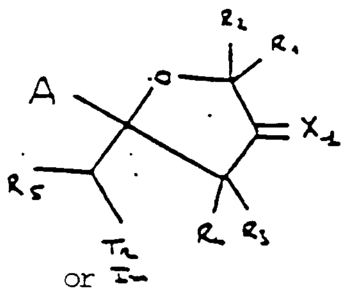

IV

in which $R_1$ to $R_5$, n, Y and A have the same meaning as in Claim 1 in which $X_1$ and $X_2$ together correspond to an oxygen atom, characterised in that a compound of formula III or IIIa in which Y, n and $R_1$ to $R_5$ have the same meaning as in Claim 1 and Z is a halogen atom, and in which $QR_6$ corresponds to a hydroxyl group and $X_1$ to a hydrogen atom, is oxidised, and in that, when the starting material is the compound IIIa, an unsubstituted imidazole or triazole ring is then attached.

**14.** Process for the preparation of compounds of formula IV in which $X_1$ and $X_2$ together correspond to a sulphur atom and Y, n and $R_1$ to $R_5$ have the same meaning as in Claim 1, characterised in that the compound of the formula IV in which $X_1$ and $X_2$ together correspond to an oxygen atom is subjected to a thionation.

**15.** Process for the preparation of the compounds of formula V, characterised in that the compounds of formula IV or IVa

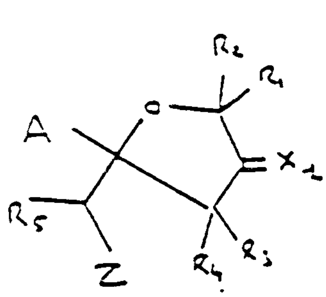

IVa

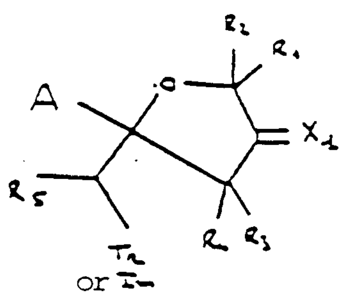

IV

in which $X_1$ and $X_2$ together correspond to an oxygen or sulphur atom doubly bonded to the tetrahydrofuran are reacted with a compound of formula $HQR_6$, where Q corresponds to O or S, in the presence of an acid catalyst and in a suitable solvent, which can be the alcohol or thiol itself or an inert

solvent; if the starting material is the compound of formula IVa, the compound of formula Va is obtained, and an imidazole or triazole ring is then grafted onto the compound of formula Va.

**16.** Process for the preparation of the compounds of formula II in which $R_1$ to $R_5$, n, Y and A have the same meaning as in Claim 1 where $X_1$ and $X_2$ correspond to a halogen atom, preferably chlorine or bromine, characterised in that a compound of formula III or IIIa in which Y, n, $R_1$ to $R_5$ and A have the same meaning as in Claim 1 and Z is a halogen atom, and in which $QR_6$ corresponds to a hydroxyl group and $X_1$ to a hydrogen atom, is oxidised and in that phosphorous pentachloride is then added to the ketone (compound of formula IV in which $X_1$ corresponds to an oxygen atom), optionally in the presence of an aprotic polar solvent and optionally of a quaternary ammonium halide, preferably at room temperature.

**17.** Compounds which can be used, where appropriate, as intermediates in the preparation processes of Claims 9 to 16, characterised in that they correspond to the formulae IIIc and IIIe

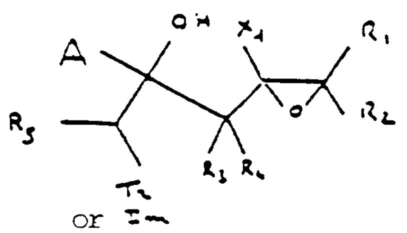

IIIc

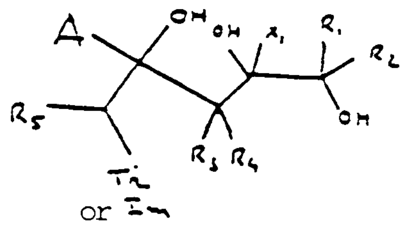

IIIe

in which $R_1$ to $R_5$, Y, A, m, n, $X_1$ and $X_2$ have any one of the meanings stated in Claim 1 or 8.

**18.** Use of the compounds according to one of Claims 1 to 8 as a fungicide, in particular in the form of fungicidal compositions containing as active substance a compound according to one of Claims 1 to 8, preferably 0.5 to 95 % by weight, this active substance being in combination with at least one agriculturally acceptable inert carrier.

**19.** Process for the control of fungal diseases of crops, characterised in that an effective dose of an active substance according to one of Claims 1 to 8 is applied, preferably at a rate of 0.005 to 5 kg/ha, and advantageously 0.01 to 0.5 kg/ha.

## Patentansprüche

**1.** Verbindungen mit Triazol- oder Imidazol-, Tetrahyrofuran- und Phenylgruppen,

dadurch gekennzeichnet, daß sie der allgemeinen Formel I entsprechen,

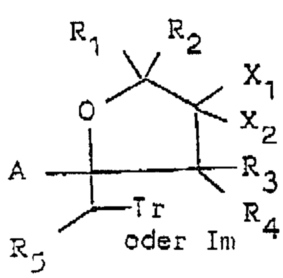

worin bedeuten:

- A eine Gruppe $(Y)_n$-Ph-$(CH_2)_m$-, worin darstellen:
- Ph eine Phenylengruppe,
- m 0 oder 1

und

Y ein Halogenatom, Cyano, Nitro, ggf. halogeniertes Alkyl oder ggf. halogeniertes Alkoxy,

n 0, 1, 2, 3, 4 oder 5,

n > 1, wobei die Substituenten Y bei n > 1 gleich oder verschieden sein können,

Tr 1,2,4-Triazol-1-yl und Im 1,3-Imidazol-1-yl,

$R_1$ bis $R_5$, die gleich oder verschieden sein können, ein Wasserstoffatom, geradkettiges oder verzweigtes Alkyl mit höchstens 6 C-Atomen, Cycloalkyl mit höchstens 6 C-Atomen, Phenyl, Benzyl oder Alkoxy mit höchstens 6 C-Atomen, wobei diese verschiedenen Gruppen ggf. mit einem oder mehreren Halogenatomen oder einer oder mehreren Alkoxygruppen mit höchstens 6 C-Atomen substituiert sind,

sowie

$X_1$ und $X_2$, die gleich oder verschieden sein können, ein Wasserstoffatom, ein Halogenatom, Alkyl mit höchstens 6 C-Atomen, Cycloalkyl mit höchstens 6 C-Atomen, Phenyl oder Benzyl, wobei diese verschiedenen Gruppen ggf. in gleicher Weise wie die Gruppen $R_1$ bis $R_5$ substituiert sind,

eine Gruppe Q-$R_6$, in der Q O oder S und $R_6$ ein Wasserstoffatom, Alkyl mit höchstens 6 C-Atomen, Cycloalkyl mit höchstens 6 C-Atomen, Phenyl, Benzyl, Benzoyl oder Acyl bedeuten, wobei diese Gruppen ggf. in gleicher Weise wie die Gruppen $R_1$ bis $R_5$ substituiert sind und gleich oder verschieden sein können, wenn $X_1$ und $X_2$ jeweils $QR_6$ oder $SR_6$ bedeuten,

zusammen eine Gruppe der Formel -Q-$R_7$-Q-, wobei Q die obige Bedeutung besitzt und $R_7$ eine zweiwertige Kohlenwasserstoffgruppe mit 2 bis 6 C-Atomen darstellt und eines dieser C-Atome durch ein Sauerstoff- oder Stickstoffatom ersetzt sein kann und die einzige zweiwertige Kohlenwasserstoffgruppe selbst, ggf. mit einem oder mehreren Halogenatomen, einer oder mehreren Hydroxygruppen, einer oder mehreren Alkylgruppen mit höchstens 6 C-Atomen oder einer oder mehreren ggf. halogenierten Alkoxygruppen mit höchstens 6 C-Atomen substituiert ist,

eine Gruppe -$NR_8R_9$, in der $R_8$ und $R_9$, die gleich oder verschieden sein können, ein Wasserstoffatom oder Alkyl mit höchstens 6 C-Atomen, Cycloalkyl mit höchstens 6 C-Atomen oder Phenyl bedeuten, wobei diese verschiedenen Gruppen ggf. in gleicher Weise wie die Gruppen $R_1$ bis $R_5$ substituiert sind, oder wobei $R_8$ und $R_9$ zusammen eine einzige zweiwertige kohlenwasserstoffgruppe mti 2 bis 6 C-Atomen bilden, bei der eines der C-Atome durch ein Sauerstoffatom, ein Schwefelatom oder ein Stickstoffatom ersetzt sein kann, und wobei diese einzige Kohlenwasserstoffgruppe ihrerseits ggf. mit einem oder mehreren Halogenatomen, einer oder mehreren Hydroxygruppen, einer oder mehreren Alkylgruppen mit höchstens 6 C-Atomen oder einer oder mehreren Alkoxygruppen mit höchstens 6 C-Atomen substituiert ist,

zusammen eine Gruppe =N-$R_{10}$, in der $R_{10}$ Alkyl mit höchstens 6 C-Atomen, Cycloalkyl mit höchstens 6 C-Atomen, eine Gruppe $OR_{11}$, bei der $R_{11}$ Alkyl darstellt, wobei diese Gruppe ggf. in gleicher Weise wie die Gruppen $R_1$ bis $R_5$ substituiert sind, oder Hydroxy bedeutet,

eine Gruppe $N_3$ mit der Maßgabe, daß $X_2$ von $N_3$ verschieden ist,

oder

$X_1$ und $X_2$ zusammen ein Sauerstoffatom =O oder ein Schwefelatom =S,

mit den allgemeinen Maßgaben, daß $X_1$ und $X_2$ nicht gleichzeitig Wasserstoffatome, Alkyl mit höchstens 6 C-Atomen, Cycloalkyl mit höchstens 6 C-Atomen, Phenyl oder Benzyl darstellen, und, wenn $X_1$ und $X_2$ verschieden sind und einer dieser beiden Substituenten -$OR_6$ bedeutet, $R_6$ ausschließlich in diesem Fall nur Benzoyl oder Acyl bedeuten kann,

sowie ihre Salze.

**2.** Verbindungen nach Anspruch 1, dadurch gekennzeichnet, daß m 0, n 1, 2 oder 3 und Y ein Halogenatom bedeuten.

**3.** Verbindungen nach den Ansprüchen 1 und 2, dadurch gekennzeichnet, daß n 1 oder 2 und Y ein o- und/oder p-ständiges Halogenatom bedeuten.

**4.** Verbindungen nach Anspruch 3, dadurch gekennzeichnet, daß Y ein Chlor- oder Bromatom ist.

**5.** Triazolverbindungen nach einem der Ansprüche 1 bis 4, dadurch gekennzeichnet, daß sie der Formel II entsprechen,

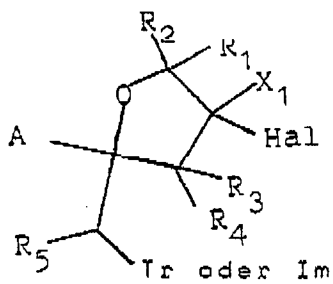

II,

wobei bedeuten:

| | |
|---|---|
| Y, m und n | eine der für die Verbindung der Formel I angegebenen Bedeutungen, |
| $R_1$ bis $R_5$ | ein Wasserstoffatom oder eine Alkyl- oder Cycloalkylgruppe mit höchstens 6 C-Atomen, |
| Hal | ein Halogenatom |
| | und |
| $X_1$ | ein Halogenatom oder ein Wasserstoffatom. |

**6.** Verbindungen nach Anspruch 5, dadurch gekennzeichnet, daß Hal Chlor oder Brom und $X_1$ Chlor oder Brom bedeuten.

**7.** Triazolverbindungen nach einem der Ansprüche 1 bis 4, dadurch gekennzeichnet, daß sie der Formel III entsprechen,

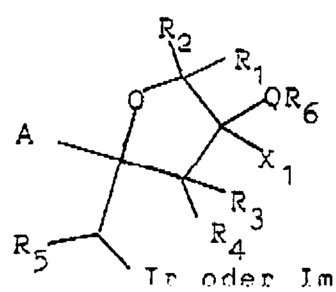

III,

wobei bedeuten:

| | |
|---|---|
| Y, n und m | eine der für die Verbindung der Formel I angegebenen Bedeutungen, |
| $R_1$ bis $R_5$ | ein Wasserstoffatom oder eine Alkyl- oder Cycloalkylgruppe mit höchstens 6 C-Atomen, |
| $X_1$ | ein Wasserstoffatom oder eine Alkyl- oder Cycloalkylgruppe mit höchstens 6 C-Atomen, |
| Q | dasselbe wie in Formel I |

und

$R_6$ ein Wasserstoffatom oder eine Alkyl- oder Cycloalkylgruppe mit höchstens 6 C-Atomen.

**8.** Verbindungen nach Anspruch 7, dadurch gekennzeichnet, daß $R_1$ bis $R_5$ ein Wasserstoffatom oder Alkyl oder Cycloalkyl mit höchstens 6 C-Atomen und $R_6$ Alkyl oder ggf. substituiertes Cycloalkyl mit höchstens 6 C-Atomen bedeuten.

**9.** Verfahren zur Herstellung der Verbindungen der Formel II, in der $R_1$ bis $R_5$, A, Y, n und $X_1$ die gleiche Bedeutung wie in Anspruch 1 besitzen und Hal ein Halogenatom darstellt,

gekennzeichnet durch folgende Stufen:

Stufe a) Umsetzung eines Halogenketons der Formel IIa,

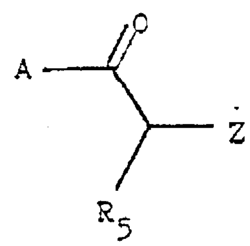

IIa,

wobei Y, n und $R_5$ dasselbe wie bei den Verbindungen der Formel I

und

Z ein Halogenatom bedeuten,

mit einer Organometallverbindung der Formel IIb

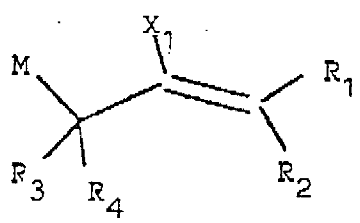

IIb

zu einer Verbindung der Formel IIc

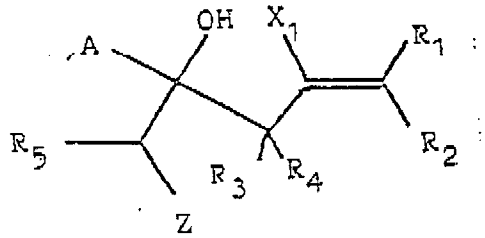

IIc,

Stufe b) anschließende Umsetzung der Verbindung der Formel IIc mit einem nichtsubstituierten Imidazol oder Triazol in Gegenwart einer anorganischen oder organischen Base sowie in einem geeigneten Lösungsmittel,

Stufe c) Umsetzung der erhaltenen Verbindung der Formel IId

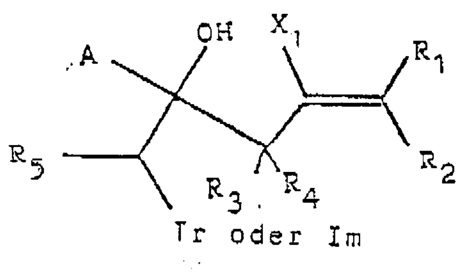

IId

mit einem Halogen oder Halogengemisch in einem inerten Lösungsmittel unter Erhalt einer Verbindung der Formel IIe

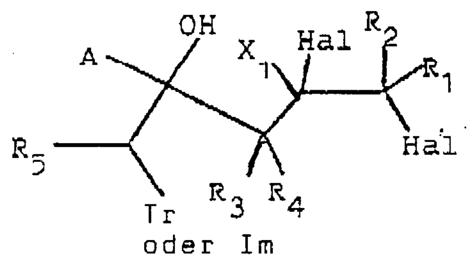

IIe

und

Stufe d) Cyclisierung der Verbindung der Formel IIe in Gegenwart einer anorganischen oder organischen Base zur Verbindung der Formel II.

**10.** Verfahren zur Herstellung der Verbindungen der Formel II, wobei

$R_1$ bis $R_5$, Y, n, A und $X_1$ dasselbe wie in Anspruch 1 bedeuten und Hal ein Halogenatom darstellt,

gekennzeichnet durch Umsetzung der Verbindung der Formel IIc nach Anspruch 9 mit einem Halogen oder Halogengemisch in einem inerten Lösungsmittel unter Erhalt der Verbindung der Formel IIf,

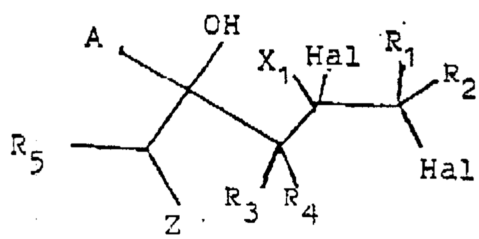

IIf ,

Cyclisierung der Verbindung der Formel IIf in Gegenwart einer organischen oder anorganischen Base zu einer Verbindung der allgemeinen Formel IIg

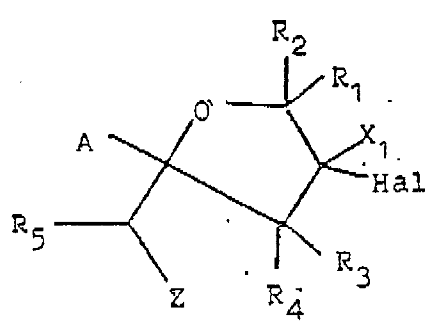

IIg

und

Verknüpfung mit einer nichtsubstituierten Triazol- oder Imidazolgruppe in Gegenwart einer organischen oder anorganischen Base und in einem geeigneten Lösungsmittel.

**11.** Verfahren zur Herstellung der Verbindungen der Formeln III oder VI,

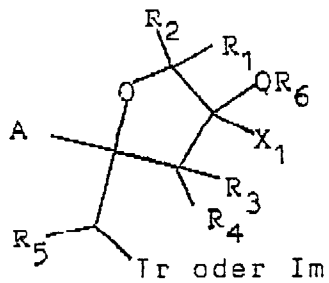

III

oder

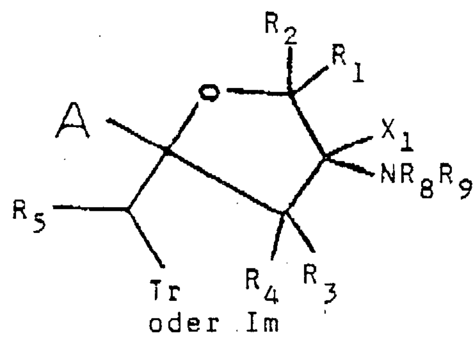

VI,

worin bedeuten:

$R_1$ bis $R_6$, Y, n, A und $X_1$ dasselbe wie in Anspruch 1

und

Q ein Sauerstoff- oder ein Schwefelatom,

gekennzeichnet durch

Umsetzung einer Verbindung der Formel II oder einer Verbindung der Formel IIg mit einem heteroatomischen Nucleophil der Formel $R_6$-Q-E, worin $R_6$ und Q dasselbe wie bei den Verbindungen der Formel I und E ein Kation bedeuten,

in Gegenwrt einer Base und ggf. eines Phasenübertragungskatalysators

sowie, wenn von der Verbindung der Formel IIg ausgegangen wird, Verknüpfung mit einem unsubstituierten Imidazol oder Triazol.

**12.** Verfahren zur Herstellung der Verbindungen dar Formel III, in der $R_6$ ggf. mono- oder polyhalogeniertes Alkyl mit höchstens 6 C-Atomen darstellt, gekennzeichnet durch Umsetzung einer Verbindung der Formel III oder einer Verbindung der Formel IIIa,

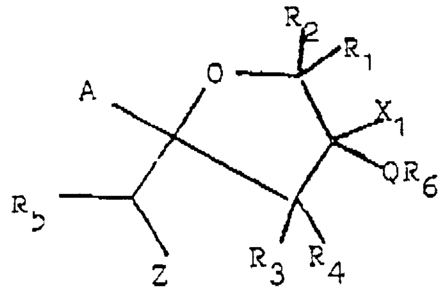

IIIa,

worin $R_6$ ein Wasserstoffatom darstellt,

mit einem (ggf. mono- oder polyhalogenierten) Alkylhalogenid in Gegenwart einer organischen oder anorganischen Base

sowie, wenn von einer Verbindung der Formel IIIa ausgegangen wurde, anschließende Verknüpfung mit einer Triazol- oder Imidazolgruppe.

**13.** Verfahren zur Herstellung der Verbindungen der Formel IV,

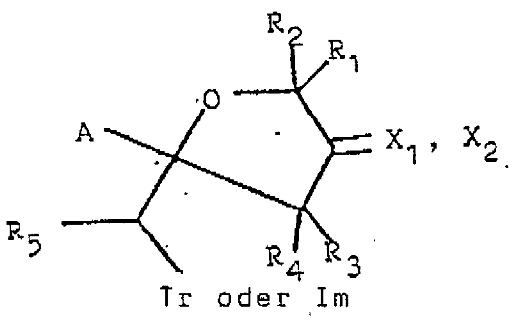

IV,

worin bedeuten:

$R_1$ bis $R_5$, n, Y und A dasselbe wie in Anspruch 1 und
$X_1$ und $X_2$ zusammen ein Sauerstoffatom,

gekennzeichnet durch Oxidation einer Verbindung der Formel III oder der Formel IIIa, worin Y, n und $R_1$ bis $R_5$ dasselbe wie in Anspruch 1 bedeuten und $QR_6$ eine Hydroxygruppe und $X_1$ ein Wasserstoffatom darstellen,

sowie, wenn von einer Verbindung der Formel IIIa ausgegangen wurde, anschließende Verknüpfung mit einer nichtsubstituierten Imidazol- oder Triazolgruppe.

**14.** Verfahren zur Herstellung der Verbindungen der Formel IV, worin Y, n und $R_1$ bis $R_5$ dasselbe wie in Anspruch 1 bedeuten und $X_1$ und $X_2$ zusammen ein Schwefelatom darstellen, gekennzeichnet durch Thionierung einer Verbindung der Formel IV, in der $X_1$ und $X_2$ zusammen ein Sauerstoffatom darstellen.

**15.** Verfahren zur Herstellung der Verbindungen der Formel V, gekennzeichnet durch Umsetzung von Verbindungen der Formeln IV

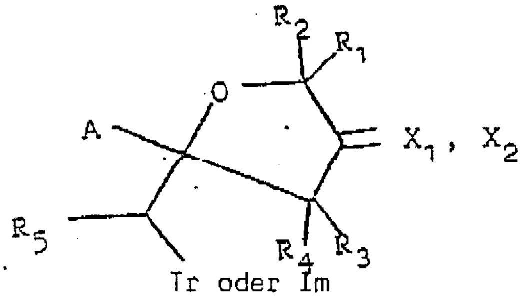

IV

oder IVa

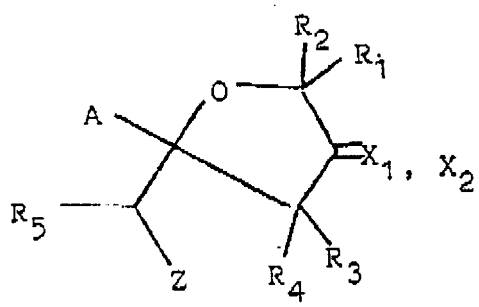

IVa,

worin

$X_1$ und $X_2$ zusammen ein über eine Doppelbindung an das Tetrafurangerüst gebundenes Sauerstoff- oder Schwefelatom bedeuten,

mit einer Verbindung der Formel $HQR_6$, worin Q O oder S bedeutet, in Gegenwart eines sauren Katalysators sowie in einem geeigneten Lösungsmittel, wobei der Alkohol oder das Thiol selbst oder ein inertes Lösungsmittel verwendbar sind,

wobei, wenn von einer Verbindung der Formel IVa ausgegangen wurde, die Verbindung der Formel Va erhalten wird, die anschließend mit einer Imidazol- oder Triazolgruppe verknüpft wird.

**16.** Verfahren zur Herstellung der Verbindungen der Formel II, in der $R_1$ bis $R_5$, n, Y und A dasselbe wie in Anspruch 1 bedeuten und $X_1$ und $X_2$ ein Halogenatom, vorzugsweise Chlor oder Brom, darstellen,

gekennzeichnet durch Oxidation einer Verbindung der Formel III oder der Formel IIIa, worin Y, n, $R_1$ bis $R_5$ und A dasselbe wie in Anspruch 1 bedeuten und

Z ein Halogenatom,

$QR_6$ eine Hydroxygruppe

und

$X_1$ ein Wasserstoffatom darstellen,

und anschließende

Umsetzung des erhaltenen Ketons (Verbindung der Formel IV, worin $X_1$ ein Sauerstoffatom darstellt) mit Phosphorpentachlorid, ggf. in Gegenwart eines inerten aprotischen Lösungsmittels sowie ggf. eines quaternären Ammoniumhalogenids, vorzugsweise bei Raumtemperatur.

**17.** Verbindungen, die ggf. als Zwischenprodukte in den Herstellungsverfahren der Ansprüche 9 bis 16 verwendbar sind, dadurch gekennzeichnet, daß sie die Formeln IIIc und IIIe aufweisen,

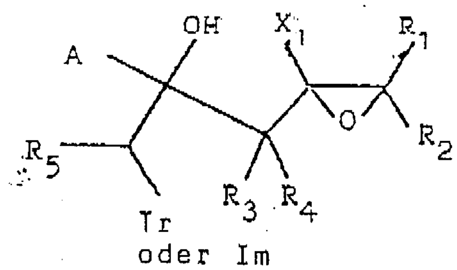

IIIc

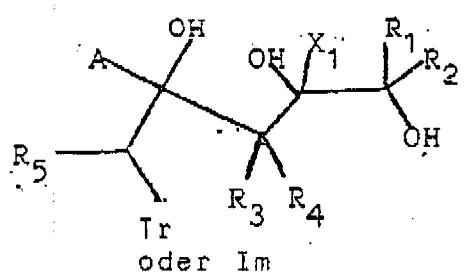

(IIIe),

worin $R_1$ bis $R_5$, $Y_1$ A, m, n und $X_1$ und $X_2$ eine der in Anspruch 1 oder 8 angegebenen Bedeutungen aufweisen.

**18.** Verwendung der Verbindungen nach einem der Ansprüche 1 bis 8 als Fungicide, insbesondere in Form fungicider Zusammensetzungen, die als Wirkstoff eine Verbindung nach einem der Ansprüche 1 bis 8 enthalten, vorzugsweise in einer Menge von 0,5 bis 95 Masse-%, wobei dieser Wirkstoff zusammen mit mindestens einem inerten, agrikulturchemisch geeigneten Trägerstoff vorliegt.

**19.** Verfahren zur Bekämpfung von Pilzerkrankungen bei Kulturpflanzen, gekennzeichnet durch Anwendung einer wirksamen Dosis eines Wirkstoffs nach einem der Ansprüche 1 bis 8, vorzugsweise in einer Dosis von 0,005 bis 5 kg/ha und vorteilhaft in einer Dosis von 0,01 bis 0,5 kg/ha.